# EUROPÄISCHE PATENTSCHRIFT

(11) **EP 3 328 358 B1**
(45) Veröffentlichungstag und Bekanntmachung des Hinweises auf die Patenterteilung: **23.11.2022**
(21) Anmeldenummer: 16759666.7
(22) Anmeldetag: 25.07.2016
(51) Int. Cl.: A61K 31/5575, A61K 9/00, A61K 47/10, A61K 47/12, A61K 9/08, A61P 9/00

(54) **KONZENTRAT ENTHALTEND ALPROSTADIL**
CONCENTRATE CONTAINING ALPROSTADIL
CONCENTRÉ CONTENANT DE L'ALPROSTADIL

(30) Priorität: 27.07.2015 AT 506662015
(43) Veröffentlichungstag der Anmeldung: 06.06.2018
(73) Patentinhaber: Gebro Holding GmbH, 6391 Fieberbrunn (AT)
(72) Erfinder: HESSE, Ernst, 6391 Fieberbrunn (AT); HANSBAUER, Bernhard, 5061 Elsbethen-Glasenbach (AT); HÄUSLER, Franz, 83435 Bad Reichenhall (DE)
(74) Vertreter: Wildhack & Jellinek
(86) Internationale Anmeldenummer: PCT/AT2016/060020
(87) Internationale Veröffentlichungsnummer: WO 2017/015692

(56) Entgegenhaltungen:
- EP-A2- 0 407 148
- WO-A1-95/11683
- CN-A- 102 048 687

## Beschreibung

Die Erfindung betrifft eine flüssige pharmazeutische Zusammensetzung als Konzentrat zur Herstellung einer Infusionslösung, enthaltend Alprostadil (Prostaglandin E1) gemäß dem Oberbegriff des Anspruchs 1.

Alprostadil (PGE1) ist ein im Europäischen Arzneibuch beschriebener Wirkstoff aus der Gruppe der Prostaglandin Analoga. Es ist identisch mit dem körpereigenen Prostaglandin E1. Seine hauptsächlichen physiologischen Effekte bestehen in einer Vasodilatation und in einer Hemmung der Thrombozytenaggregation. Aus diesem Wirkprofil leiten sich als die wichtigsten Indikationen die Behandlung erektiler Dysfunktion, die Behandlung peripherer arterieller Verschlusskrankheiten und die kurzzeitige Behandlung bestimmter Herzfehler beim Neugeborenen ab. Als pharmazeutische Darreichungsformen stehen dafür hauptsächlich parenterale, zur Behandlung der erektilen Dysfunktion auch topische Arzneiformen zur Verfügung.

Alprostadil ist ein chemisch außerordentlich instabiler Wirkstoff. Als wichtigste Abbaureaktion nennt der Kommentar des Europäischen Arzneibuchs die Abspaltung von Wasser zu Prostaglandin A₁ (PGA1). Dieses Abbauprodukt wird in der Monographie des Europäischen Arzneibuchs als Verunreinigung A bezeichnet. Sie kann in einer Isomerisierungsreaktion weiter zu Prostaglandin B₁ reagieren, das in der Monographie des Europäischen Arzneibuchs als Verunreinigung B bezeichnet wird. Insgesamt nennt das Europäische Arzneibuch elf Verunreinigungen mit ihrer jeweiligen Molekülstruktur. Generell ist die Stabilität des Alprostadils in nicht-wässrigen Lösungsmitteln, insbesondere in Alkoholen oder in lipophilen Lösungsmitteln, besser als in reinem Wasser. Gemäß dem Kommentar des Europäischen Arzneibuchs findet die Startreaktion des Abbaus, nämlich die Wasserabspaltung zu PGA1, besonders leicht unter sauren oder basischen Bedingungen statt. In wässrigen oder wasserhaltigen Lösungen kann die Stabilität aber auch bei relativ neutralen pH-Werten durch die eingesetzten Pufferkomponenten oder durch Isotonisierungszusätze verschlechtert werden. So ist die Stabilität einer Lösung von Alprostadil in reinem Wasser besser als die einer Lösung von Alprostadil in physiologischer, 0,9 %iger NaCI-Lösung und deutlich besser als von Alprostadil in einem Phosphatpuffer gleicher Tonizität und gleichen pH-Wertes. Da die Carboxylfunktion des Alprostadils selbst einen negativen Einfluss auf die Stabilität der Substanz hat, verläuft die Zersetzungsreaktion zumindest teilweise autokatalytisch.

Wegen der Instabilität des Wirkstoffes, insbesondere wegen seiner Neigung zur autokatalytischen Zersetzung, und, weil es wegen der Empfindlichkeit gegenüber den üblichen Puffersubstanzen nicht möglich ist, den pH-Wert durch einen Puffer auf einen für die Stabilität optimalen Wert einzustellen, ist es nicht möglich, gebrauchsfertige parenterale Zubereitungen herzustellen, die eine für die Vermarktung ausreichende Lagerstabilität aufweisen. Alprostadil zur parenteralen Anwendung wird deshalb ausschließlich in Form von Konzentraten angeboten, die kurz vor der Anwendung durch Auflösung in einer geeigneten sterilen Lösung (z.B. physiologische Kochsalzlösung) zur gebrauchsfertigen Infusionslösung verdünnt bzw. rekonstituiert werden. Wiederum wegen der Instabilität in wässrigem Milieu, aber auch, weil Alprostadil in Wasser für die Herstellung eines Konzentrats nicht ausreichend löslich ist, werden als Konzentrate im Wesentlichen entweder Lyophilisate oder nicht wässrige Lösungen verwendet.

In WO 95/11683 ist beispielsweise eine lyophilisierte PGE₁ -Formulierung offenbart.

Lyophilisate sind feste, pulverförmige Konzentrate, die durch Gefriertrocknung des Wirkstoffes zusammen mit stabilisierenden Hilfsstoffen hergestellt werden. Der Herstellprozess ist aufwändig und teuer; das Lyophilisat wird entweder in Vials oder anwenderfreundlicher in Zweikammerfertigspritzen oder Doppelkammerkarpulen verpackt und kann bei Raumtemperatur gelagert werden. Als stabilisierender Hilfsstoff wird hauptsächlich alpha-Cyclodextrin (Alfadex) verwendet. Handelsbezeichnungen solcher Produkte sind z.B. Caverject^{®} (Pfizer, Pharmacia), Prostavasin^{®} (UCB Pharma) oder Viridal^{®} (UCB Pharma). Die CN 102688203 beschreibt ein Lyophilisat enthaltend Alprostadil bei dem zur Stabilisierung eine Mischung von Dextran und Hydroxypropyl-beta-Cyclodextrin verwendet wird.

Als nicht wässrige Lösungen werden im Wesentlichen Lösungen des Wirkstoffes in absolutem Ethanol verwendet. Diese ethanolischen Konzentrate müssen ständig bei tiefen Temperaturen von 2 bis 8 °C gelagert werden. Die Aufrechterhaltung der Kühlkette und der Nachweis ihrer Einhaltung stellen für die Hersteller und Anwender der Produkte einen erheblichen Aufwand dar und verursachen hohe Kosten. Trotzdem beträgt die Verwendungsfrist der Zubereitungen typischerweise nur 18 Monate. Handelsbezeichnungen dieser Produkte sind z.B. Pridax^{®} (Gebro, Pharmore), Alprestil^{®} (Gebro), Miniprog^{®} (Pfizer, Pharmacia) oder Alprostadil "PINT"^{®} (Pint Pharma).

In der Literatur sind auch ölige und liposomale Zubereitungen und Konzentrate beschrieben. Letztere werden in Japan z.B. unter den Markenbezeichnungen Liple^{®} (Tanabe) und Palux^{®} (Taisho) auch vermarktet.

In EP 0 407 148 ist beispielsweise eine ölige Emulsion eines Wirkstoffs offenbart, der PGE₁-Aktivität aufweist.

Auch CN 102048687 A offenbart eine ölige Emulsion von Alprostadil.

Die Stabilität halbfester Zubereitungen ist auch unter günstigsten Bedingungen auf wenige Monate beschränkt, was den praktischen Einsatz solcher Produkte insbesondere in der Indikation der erektilen Dysfunktion stark limitiert. Als topische Zubereitungen werden reine Lipogele beschrieben, die natürliche Öle, Fettsäuren, Fettalkohole oder synthetische Lipide, wie z.B. Isopropylmyristat, enthalten und die zusätzlich mit Verdickungsmitteln, wie z.B. Poloxameren, verdickt werden können. Beschrieben sind derartige Lipogele z.B. in KR 20010011625 und in JP 0383925.

Weitere Darreichungsformen, z.B. feste Stäbchen zur Anwendung in der Harnröhre werden z.B. unter dem Markennamen Muse^{®} von der Firma Abbott angeboten.

Für die vorliegende Erfindung stellt die Lösung des Wirkstoffes in absolutem Ethanol (z.B. Pridax^{®} 20 µg/ml Konzentrat zur Herstellung einer Infusionslösung) den nächstkommenden Stand der Technik dar. Der Hauptvorteil der ethanolischen Lösung besteht darin, dass sie keine weiteren Hilfsstoffe, insbesondere kein alpha-Cyclodextrin, gegen das es toxikologische Bedenken gibt, enthält.

Ein weiterer Vorteil der ethanolischen Lösung ist, dass sie einfacher zu einer infusionsfertigen Lösung verarbeitet werden kann als ein Lyophilisat. Das Lyophilisat muss zunächst in einem ersten Schritt im Vial mit einem geeigneten Lösungsmittel rekonstituiert werden; anschließend muss diese Lösung in einem zweiten Schritt der Infusionslösung zugesetzt werden. Demgegenüber kann die Ampulle direkt in einem Schritt zu einer Infusionslösung gegeben werden. Diese Vereinfachung des Handlings reduziert auch das Risiko von Manipulationsfehlern und das Risiko, Keime einzuschleppen.

Ein weiterer Vorteil der ethanolischen Lösung ist die sehr einfache Zusammensetzung der Formulierung, bestehend vorzugsweise ausschließlich aus dem Wirkstoff und dem Lösungsmittel. Sie ermöglicht eine im Vergleich zu einem Lyophilisat viel einfachere und preisgünstigere Herstellung.

Ein weiterer Vorteil ist, dass die einfache Zusammensetzung der Formulierung die Analytik der Zubereitung wesentlich erleichtert. Wegen der geringen Dosierung des Wirkstoffes müssen im Rahmen der Qualitätskontrolle Verunreinigungen bis in einen Konzentrationsbereich von 10 ng/ml quantifiziert werden. Bei so niedrigen Konzentrationen ist es ein großer Vorteil, wenn die Zubereitung ohne Aufarbeitung und ohne Abtrennung von Hilfsstoffen direkt analysierbar ist.

Der Hauptnachteil der rein ethanolischen Lösung besteht in ihrer geringen chemischen Stabilität mit folgenden nachteiligen Auswirkungen:
- Es müssen hohe Konzentrationen von Abbauprodukten im Fertigprodukt in Kauf genommen werden (z.B. 6 % PGA1)
- Die Laufzeit der Fertigprodukte ist begrenzt (z.B. 18 Monate)
- Die Lagerung der Fertigprodukte muss kühl erfolgen, eine permanente Kühlkette ist aufrecht zu erhalten (2 - 8 °C)
- Die Standzeit bei Raumtemperatur, die im Zuge des Herstellprozesses unvermeidlich ist, muss auf wenige Stunden begrenzt werden.

Ein weiterer Nachteil der ethanolischen Lösung besteht darin, dass der als Lösungsmittel verwendete Ethanol den Patienten bei der Anwendung des Arzneimittels direkt in die Blutbahn infundiert werden muss. Obwohl die verwendete Ethanolmenge so gering ist, dass sich normalerweise ein Blutalkoholspiegel, der kleiner als 0,2 ‰ ist, einstellt, ist die systemische Applikation von Alkohol per se unerwünscht. Bedenken bestehen beispielsweise in Hinblick auf bestimmte Patientengruppen, wie z.B. Alkoholabhängige oder Menschen mit geringer Aktivität des Alkoholdehydrogenase Enzyms. Außerdem kann es durch Alkohol zu einer lokalen Reizung der Gefäßendothelien im Bereich der Einstichstelle kommen. Dies gilt analog auch für andere nicht-wässrige bzw. organische Lösungsmittel; auch hier ist es vorteilhaft die zugeführte Menge zu beschränken.

Ein weiterer Nachteil z.B. einer alkoholischen Lösung besteht darin, dass deren Abfüllung in Ampullen sehr schwierig und aufwändig ist. Ampullen werden nach der Abfüllung mittels einer offenen Gasflamme, die den Ampullenkopf zuschweißt, verschlossen. Befindet sich in der Ampulle z.B. Ethanol, enthält der Gasraum in der Ampulle oberhalb der Flüssigkeit wegen des hohen Dampfdrucks des Ethanols große Mengen an gasförmigem Ethanol. Beim Kontakt mit der Gasflamme kann sich der Ethanoldampf entweder als Stichflamme entzünden oder langsam verbrennen, wobei die Verbrennung die Glastemperatur an der Schweißstelle erhöht. Beides führt zu nicht oder nicht ordnungsgemäß verschlossenen Ampullen. Die Ampullen mit der ethanolischen Alprostadillösung können daher nur verschlossen werden, wenn die abzufüllende Lösung auf eine Temperatur von unter - 20 °C gekühlt und so der Dampfdruck des Ethanols reduziert wird. Trotzdem ist der Ausschuss bei der Abfüllung (bis zu 10 %) deutlich höher als bei der Abfüllung einer normalen, wässrigen Lösung (< 3 %).

Aufgabe der vorliegenden Erfindung ist es damit, eine flüssige Zusammensetzung von Alprostadil der eingangs erwähnten Art zu schaffen, die, insbesondere zusätzlich zu den bisherigen Vorteilen und unter Vermeidung der Nachteile der ethanolischen Lösung, die physiologische Belastung bei der Anwendung verringert, einfach und kostengünstig hergestellt werden kann und vor allem einfacher in Ampullen abgefüllt werden kann, die aber weiterhin einfach in der Anwendung ist, die unkompliziert zu analysieren ist, und die gleichzeitig eine verbesserte Stabilität und eine längere Haltbarkeit aufweist und eine geringere Konzentration an Abbauprodukten enthält, und ein weniger strenges Kühlregime bei der Herstellung, beim Transport und bei der Lagerung erfordert.

Diese Aufgabe wird durch die kennzeichnenden Merkmale von Anspruch 1 gelöst. Dabei ist erfindungsgemäß vorgesehen, dass das organische Lösungsmittel ausgewählt ist aus der Gruppe von Propylenglycol, 1,3 Butandiol, Ethylacetat, Ethanol 96 % PhEur oder Wasserfreiem Ethanol PhEur bzw. absoluter Ethanol oder Mischungen davon, dass der Gesamt-Wasseranteil im Lösungsmittelgemisch im Bereich von 8 bis 85 Gew.% H₂O, bezogen auf die Gesamtmenge des Lösungsmittelgemisches, liegt und dass zumindest eine Säure bzw. eine als Säure bzw. Protonendonator wirkende Substanz als Stabilisator für den Wirkstoff enthalten ist, wobei die Säure einen pKs1-Wert von < 4,85 aufweist, und dass die Zusammensetzung frei von Salzen ist.

Wegen der aus der Praxis und der Literatur bekannten Instabilität von Alprostadil in wässriger Lösung hätte der Fachmann erwartet, dass es zu einer Verschlechterung der Stabilität führt, wenn in einer ethanolischen Alprostadil-Lösung der Ethanol sukzessive durch Wasser ersetzt wird. Diese Verschlechterung sollte umso ausgeprägter sein, je höher der Wasseranteil ist.

Überraschenderweise wurde allerdings gefunden, dass die erfindungsgemäße, Wasser enthaltende Zusammensetzung im Vergleich zum Stand der Technik (Pridax^{®} 20 µg/ml Konzentrat zur Herstellung einer Infusionslösung), also zu der wasserfreien und säurefreien alkoholischen Lösung, und auch im Vergleich zu einer säurefreien Lösung mit gleichem Wassergehalt, eine wesentlich bessere Lagerstabilität aufweist. Insbesondere ist in Gegenwart der sauren Komponente die Bildung des Hauptabbauproduktes PGA1 deutlich verlangsamt. Dieser Stabilisierungseffekt ist so ausgeprägt, dass die erfindungsgemäße Zubereitung geeignet ist, alle Zielsetzungen der Erfindung zu erreichen. Das analytische Verfahren für die Zubereitung wird durch den Zusatz von Wasser und der sauren Komponente nicht komplizierter.

Aus der Literatur (Vieillard et al., Pharmaceut Anal Acta 2013, 4:4, 4.00-1488 Alprostadil Kommentar zur Ph. Eur. 4.00) ist bekannt, dass der Wirkstoff Alprostadil sehr empfindlich gegenüber Basen, Säuren und Oxidationsmitteln ist, wobei insbesondere Basen und Säuren die Abspaltung von Wasser zum Hauptabbauprodukt PGA1 beschleunigen. Wegen der geringen Dosierung des Alprostadils in der Konzentratlösung von 20 µg/ml bis 500 µg/ml, ist damit zu rechnen, dass bereits kleine Mengen der oben genannten Verunreinigungen zu einer Verschlechterung der Stabilität führen. Wegen ihres ubiquitären Vorkommens ist eine konsequente Vermeidung dieser Verunreinigungen mit dem derzeitigen Stand der Technik, allein durch die Prozessführung bzw. die Reinheit von Packmitteln und Lösungsmitteln nicht möglich bzw. würden solche Technologien die Herstellkosten soweit erhöhen, dass das Produkt zu keinem marktüblichen Preis mehr angeboten werden könnte.

Völlig überraschend wurde nun entgegen der in der Fachliteratur vertretenen Meinung gefunden, dass sehr geringe Mengen von organischen oder anorganischen Säuren bei wässrig/alkoholischen Lösungen nicht zu der zu erwartenden Verschlechterung der Stabilität führen, sondern im Gegenteil zu einer wesentlichen Verbesserung. Unter sehr geringen Mengen von organischen oder anorganischen Säuren sind im Sinne der Erfindung Mengen zu verstehen, die zu einer Säurekonzentration von 500 µg/ml oder kleiner führen. Da nach bisherigem Stand des Wissens durch den Wasserzusatz sogar eine Stabilitätsverschlechterung zu erwarten gewesen wäre, war dieser Stabilisierungseffekt gänzlich unerwartet.

Die Verbesserung der Stabilität durch den Zusatz einer organischen Säure konnte sowohl für die wasserfreie, aber auch überraschend für die erfindungsgemäße wasserhaltige alkoholische Alprostadillösung beobachtet werden. Die für die Stabilisierung benötigte Säuremenge ist unabhängig von der Anwesenheit bzw. von der Menge des anwesenden Wassers. In Gegenwart der sauren Komponente führt zwar die Anwesenheit von steigenden Wassermengen zu einer zunehmenden Verschlechterung der Stabilität, bis zu einer Wassermenge von ca. 85 % ist aber die durch den Säurezusatz erzielte Stabilitätsverbesserung in der Lage, die durch den Wasserzusatz verursachte Stabilitätsverschlechterung zu kompensieren.

So weist beispielsweise eine Alprostadillösung, die als Lösungsmittel eine Mischung von 50 Gew.-% Wasser und 50 Gew.-% Ethanol und als saure, stabilisierende Komponente Äpfelsäure enthält, eine bessere Stabilität auf als die entsprechend rein alkoholische Alprostadillösung und als die entsprechende Alprostadillösung, die als Lösungsmittel eine Mischung von 50 Gew.-% Wasser und 50 Gew.-% Ethanol enthält. Gegenüber dem Stand der Technik, der rein alkoholischen Alprostadillösung, bietet sie zudem die Vorteile, bei der Anwendung die physiologische Belastung mit Ethanol auf die Hälfte zu reduzieren und bei der Abfüllung in Ampullen lediglich eine Abkühlung der Lösung auf 0 °C statt auf - 20 °C zu erfordern.

Die folgenden Tabelle und Graphik stellen die Stabilität einer Lösung von 20 µg/ml Alprostadil in Abhängigkeit vom Wassergehalt einer Wasser/Ethanol-Mischung für eine Formulierung ohne Äpfelsäure und mit 20 µg/ml Äpfelsäure dar.

**Tabelle 1:**

| Restgehalt an Alprostadil (% vom Startwert) und Konzentration (% des Wirkstoffes) der Verunreinigung A nach einer Lagerung von 6 Wochen bei 25 °C für eine säurefreie Formulierung und Formulierungen mit 20 - 25 µg/ml Äpfelsäure | | | | |
|---|---|---|---|---|
| Wassergehalt | ohne Zusatz von Säure | | mit Zusatz von 20 µg Äpfelsäure | |
| | PGE1 | VUA | PGE1 | VUA |
| 0 % | 88.8* | 20,9* | 100,0 | 0,6 |
| 25% | 90,7 | 15,0 | 96,5 | 3,7 |
| 50% | 92,0 | 12,4 | 95,1* | 6,1* |
| 75% | 88,0 | 19,7 | 90,4 | 13,5 |
| 95% | 80,8 | 40,9 | 84,9 | 28,2 |

Der Vergleich der in der Tabelle mit * gekennzeichneten Werte zeigt die Überlegenheit einer 50 % ethanolischen Lösung mit Äpfelsäure gegenüber dem Stand der Technik, einer rein ethanolischen Lösung ohne Äpfelsäure.

Graphik 1:

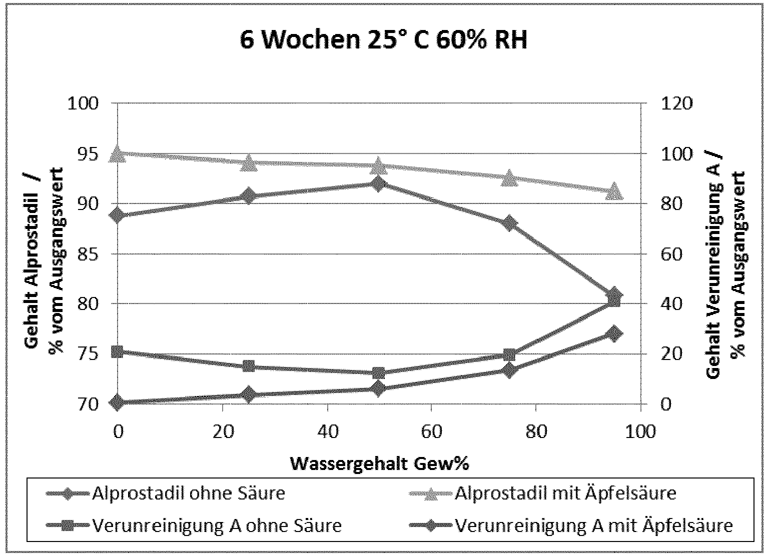

Dem Stand der Technik entsprechend wird die Konzentration des Wirkstoffes in ethanolischen Alprostadil-Lösungen in der Einheit µg/ml angegeben. In Analogie dazu wird in dieser Erfindung auch die Konzentration der Säure in der Einheit µg/ml angegeben. Eine Umrechnung in die Einheit µg/g kann jederzeit vorgenommen werden, indem durch die Dichte des Lösungsmittels dividiert wird. Bei der theoretischen Abschätzung der für eine Stabilisierung benötigten Säuremenge aus der exponentiellen Regression der erzeugten Stabilitätsdaten ergibt sich die Säuremenge in der Einheit µM, entsprechend µmol/l. Mit Hilfe des Molekulargewichts der jeweiligen Säure kann diese Konzentrationsangabe ebenfalls jederzeit in die Einheit µg/ml umgerechnet werden.

In vorteilhaften Formulierungen werden geringe Säurekonzentrationen von 0,05 - 500 µg/ml eingesetzt, vorzugsweise 0,1 - 100 µg/ml, insbesondere 5 - 50 µg/ml.

Der Begriff "pharmazeutisch annehmbares organisches Lösungsmittel" umfasst mit Wasser in den verwendeten Konzentrationsbereichen mischbare, für den Menschen physiologisch verträgliche und hinsichtlich ihrer Qualität pharmazeutisch akzeptable organische Lösungsmittel. Unter dem Begriff "organische Lösungsmittel" werden dabei auch solche organische Lösungsmittel subsummiert, die Wasser wegen ihrer Hygroskopizität als Verunreinigung enthalten, z.B. absoluter Ethanol, oder die inhärent einen gewissen Anteil an Wasser enthalten, z.B. der häufig verwendete, azeotrope 96 %ige (V/V) Ethanol.

Bei den Angaben des Gesamt-Wasseranteils bzw. des Wassergehalts in Gew.% handelt es sich vorliegend stets um den effektiven Wassergehalt, also den tatsächlichen Wasseranteil im Lösungsmittelgemisch. Allenfalls inhärent im organischen Lösungsmittel bereits enthaltenes Wasser im obigen Sinn ist dabei berücksichtigt und von den Angaben in Gew.% umfasst.

Vorteilhafterweise liegt der Gesamt-Wasseranteil im Lösungsmittelgemisch im Bereich von 10 bis 80 Gew.% H₂O, vorzugsweise von 20 bis 60 Gew.% H₂O, insbesondere von 45 bis 55 Gew.% H₂O, bezogen auf die Gesamtmenge des Lösungsmittelgemisches. In einer besonders bevorzugten Ausführungsform der Erfindung ist zur Stabilisierung einer Lösung von 20 µg/ml Alprostadil in einem Lösungsmittelgemisch enthaltend ca. 50 Gew. % Wasser und ca. 50 Gew. % Ethanol ein Säurezusatz von ebenfalls 20 µg/ml vorgesehen.

In diesem Zusammenhang ist vorteilhaft vorgesehen, dass das, insbesondere jedes, organische Lösungsmittel, in den verwendeten Konzentrationsbereichen vollständig mit Wasser mischbar ist bzw. dass das Lösungsmittelgemisch aus dem zumindest einen organischen Lösungsmittel und Wasser in den verwendeten Konzentrationsbereichen eine echte einphasige Lösung ist.

Eine vorteilhafte, weil vor allem besonders einfache, Zusammensetzung liegt vor, wenn die Zusammensetzung abschließend und ausschließlich aus Alprostadil als Wirkstoff, gelöst in einem Lösungsmittelgemisch aus zumindest einem organischen Lösungsmittel, insbesondere Ethanol, und Wasser sowie der Säure besteht, wobei der Gesamt-Wasseranteil im Lösungsmittelgemisch im Bereich von 8 bis 85 Gew.% H₂O, bezogen auf die Gesamtmenge des Lösungsmittelgemisches, liegt, und vor allem wenn sie keine weiteren Bestandteile, Exzipienten oder Additive umfasst, insbesondere wenn sie frei von weiteren Bestandteilen, Exzipienten oder Additiven, insbesondere frei von Isotonisierungszusätzen und/oder Cyclodextrinen, ist.

Ein vorteilhaftes Konzentrat liegt vor, wenn Alprostadil in einer Konzentration von 1-1000 µg/ml, vorzugsweise 5-750 µg/ml, insbesondere 20-500 µg/ml enthalten ist.

Erfindungsgemäß ist als organisches Lösungsmittel Propylenglycol, 1,3 Butandiol, Ethylacetat, , Ethanol 96 % PhEur (95.1 - 96.9 % (V/V) entsprechend 92,6 - 95,2 Gew.-%) oder Wasserfreies Ethanol PhEur (min. 99.5 % (V/V) entsprechend min. 99,2 Gew.-%) bzw. absoluter Ethanol eingesetzt werden, soweit zutreffend jeweils gemäß den Definitionen und Anforderungen der Europäischen Pharmakopöe. All diese Lösungsmittel sind in parenteralen Zubereitungen auf Grund ihres toxikologischen Profils anwendbar und der Wirkstoff Alprostadil ist darin in ausreichender Konzentration löslich. Auch Mischungen dieser Lösungsmittel sind erfindungsgemäß einsetzbar. Darüber hinaus können dem Lösungsmittel auch Lösungsvermittler wie Polyethylenglycol oder Polysorbat zugesetzt sein.

Zur analytischen Bestimmung der artifiziell zugesetzten Säure können dem Stand der Technik entsprechende analytische Methoden wie z.B. HPLC, GC, Titration oder spektroskopische Methoden herangezogen werden. Ein Nachweis des Säurezusatzes durch eine Messung des pH-Wertes ist in den erfindungsgemäßen Formulierungen wegen des Anteils an einem organischen Lösungsmittel nicht direkt möglich. In einem vereinfachten Test kann aber die Zugabe von Säure durch Verdünnung des Arzneimittels im Verhältnis von 1:25 mit einer 0,9%igen NaCl Lösung und anschließende Messung des pH-Wertes der beiden Lösungen erfolgen. Eine Senkung des pH-Wertes um mindestens 0.2 Einheiten gegenüber dem Ausgangswert der verwendeten 0,9%igen NaCl Lösung zeigt den Zusatz von Säure in der Formulierung an.

Unter den Begriffen "saure Komponente" und "Säure" werden im Sinne der Erfindung Verbindungen gemäß der Säuredefinition von Brönsted und Lowry verstanden, d.h. Substanzen, welche in der Lage sind Protonen abzugeben bzw. als Protonendonator zu fungieren. Um gegenüber dem Alprostadil als Protonendonator zu wirken, muss eine Säure einen pks-Wert aufweisen, der niedriger als 4,85, dem pks-Wert des Alprostadils, ist. Es kann sich hier sowohl um anorganische als auch organische Substanzen handeln. Die Säuren können sowohl einbasig, als auch mehrbasig sein. Vorteilhaft geeignete Säuren im Sinne der Erfindung sind solche organische und anorganische Säuren, die auf Grund ihres toxikologischen Profils in parenteralen Arzneimitteln einsetzbar sind und die sich in den erfindungsgemäßen Konzentrationen in den verwendeten Lösungsmitteln, auch bei niedrigen Temperaturen, vollständig und klar lösen. Beispiele für solche Säuren sind Salzsäure, Phosphorsäure, Essigsäure, Zitronensäure, Äpfelsäure, Milchsäure, Weinsäure oder Acetylcystein.

Als im Sinne der Erfindung besonders geeignet haben sich organische Carbonsäuren, insbesondere wegen ihres breiten Stabilisierungsoptimums mehrbasige, organische Hydroxycarbonsäuren, erwiesen. Vorzugsweise werden deshalb Säuren wie z.B. Zitronensäure, Äpfelsäure oder Weinsäure eingesetzt, besonders bevorzugt ist die Verwendung von DL-Äpfelsäure.

Verwendbar sind allerdings auch anorganische Säuren, insbesondere ausgewählt aus der Gruppe Salzsäure oder Phosphorsäure.

Stabilitätsversuche haben ergeben, dass sich eine Stabilitätsverbesserung im Wesentlichen mit all denjenigen Säuren erreichen lässt, die die hier angegebenen Bedingungen erfüllen. Eine Stabilisierung lässt sich stets sowohl daran erkennen, dass der Gehalt an Alprostadil während einer Testlagerung langsamer abnimmt, als auch daran, dass der Gehalt an Verunreinigungen, insbesondere an Verunreinigung A langsamer steigt. Die Konzentration, bei der eine maximale Stabilitätsverbesserung auftritt, sowie das Ausmaß der Stabilitätsverbesserung, hängen ungeachtet des Wassergehalts signifikant von der Art der verwendeten Säure, insbesondere von ihrem pKs1-Wert, ab, wobei die stabilisierende Wirkung in allen Fällen ein Konzentrationsoptimum durchläuft.

Keine Stabilitätsverbesserung konnte beim Zusatz saurer Puffersysteme beobachtet werden. Während z. B. der Zusatz von 50 µg/ml Essigsäure gegenüber einer rein ethanolischen Alprostadil Lösung zu einer Verbesserung der Stabilität führt, wird die Stabilität durch den Zusatz von Puffermischungen bestehend aus 50 µg/ml Essigsäure und 50 µg/ml Natriumacetat oder 25 µg/ml Essigsäure und 25 µg/ml Natriumacetat verschlechtert. Dies korrespondiert mit der Angabe aus dem Kommentar zum Europäischen Arzneibuch über die Verschlechterung der Stabilität durch den Zusatz von Phosphatpuffer.

Beim Zusatz reiner Säuren hat sich gezeigt, dass der Konzentrationsbereich der optimalen Stabilisierungswirkung sowohl bei wasserhaltigen als auch bei wasserfreien Formulierungen umso tiefer bzw. niedriger liegt und auch umso schmaler bzw. enger ist, je stärker die Säure ist. Die nachfolgende Tabelle 2 macht diesen Zusammenhang für eine Lösung von Alprostadil in wasserfreiem Ethanol in einer Konzentration von 20 µg/ml deutlich:

**Tabelle 2: Für die Stabilisierung einer Lösung von 20 µg/ml Alprostadil in wasserfreiem Ethanol geeignete Säuren und dafür geeignete Säurekonzentrationen**

| Säure | pks1-Wert | Konzentrationsbereich der Stabilisierungswirkung in µg/ml | Konzentrationsbereich der optimalen Stabilisierungswirkung in µg/ml |
|---|---|---|---|
| Salzsäure | - 7 | 0,1 - 0,8 | 0,3 - 0,7 |
| Phosphorsäure | + 2,16 | 0,5 - 15 | 1 - 10 |
| Weinsäure | + 2,98 | 2 - 150 | 5 - 50 |
| DL-Äpfelsäure | + 3,46 | 5 - 200 | 10 - 100 |
| Acetylcystein | + 3,82 | 5 - 350 | 10 - 100 |
| Essigsäure | + 4,75 | 20 - 500 | 50 - 250 |

Tabelle 2 enthält somit vorteilhafte Konzentrationen von vorteilhaft einsetzbaren Säuren, bei denen sich experimentell die besten Stabilisierungen für Alprostadil ergeben haben.

Aus den Vergleichsversuchen lässt sich weiters ableiten, dass sich vorteilhafte Stabilisierungseffekte erreichen lassen, wenn die Säure einen pKs1-Wert von < 4,85, insbesondere im Bereich von 2 bis 4,8, aufweist.

In diesem Zusammenhang hat es sich als vorteilhaft erwiesen, wenn die Säure einen pKs1-Wert von größer gleich 0 bis kleiner 4,85 aufweist und in einer Konzentration von größer gleich c min = 1,45 * e hoch (1,02 * pKs1) µM (=µmol/l) und kleiner gleich c max = 11,75 * e hoch (1,35 * pKs1) µM (=µmol/l) enthalten ist oder wenn die Säure einen pKs1-Wert von kleiner 0 aufweist und in einer Konzentration von größer gleich c min = 1,0 µM (=µmol/l) und kleiner gleich c max = 15 µM (=µmol/l) enthalten ist.

Dabei hat es sich für die Alprostadil-Stabilisierung weiters als besonders vorteilhaft erwiesen, wenn die anorganische oder organische Säure einen pKs1-Wert von größer gleich 2 bis kleiner gleich 4,8 aufweist und in einer Konzentration von 0,5 bis 500 µg/ml, insbesondere von 1 bis 250 µg/ml, enthalten ist.

Die Abfüllung des erfindungsgemäßen Konzentrats erfolgt in geeignete Primärpackmittel. Als Primärpackmittel werden in diesem Zusammenhang Packmittel verstanden, welche in direktem Kontakt mit dem Arzneimittel stehen. Sie müssen einerseits den Anforderungen der einschlägigen Arzneibücher entsprechen und müssen darüber hinaus so ausgewählt werden, dass sie möglichst wenig für die Stabilität des Inhalts schädliche Substanzen, wie z.B. basische Verunreinigungen, abgeben. Geeignete Materialien, aus denen für empfindliche Parenteralia einsetzbare Primärpackmittel bestehen, sind dem Fachmann bekannt. Denkbar sind beispielsweise, aber nicht ausschließlich Röhrenglas oder Hüttenglas der hydrolytischen Klasse I oder der hydrolytischen Klasse II, mit Innenvergütung. Ebenso anwendbar sind beispielsweise, aber nicht ausschließlich Kunststoffe wie COP, COC, PP, PE, HDPE, Teflon, PA.

Bevorzugte Primärbehältnisse sind Glasampullen oder Durchstechflaschen (Vials), bestehend aus Röhrenglas der hydrolytischen Klasse I. Als primäres Verschlussmaterial für Durchstechflaschen kommen Stopfen aus Brombutyl-Kautschuk oder Chlorbutyl-Kautschuk, vorzugsweise beschichtet, in Frage. Weitere mögliche Primärbehältnisse sind beispielsweise, aber nicht ausschließlich, Karpulen oder Fertigspritzen.

Daraus ergibt sich, dass eine gute Stabilisierung der Zusammensetzung bzw. von Alprostadil bei einer Kit-of-parts-Kombination aus der erfindungsgemäßen Zusammensetzung abgefüllt bzw. enthalten in einer dicht verschlossenen Glasampulle, Glasvial, Karpule oder Fertigspritze, vorliegt.

Die erfindungsgemäße pharmazeutische Zusammensetzung ist vorteilhafterweise ein Konzentrat für die Erstellung einer parenteral zu verabreichenden Infusionslösung, welches insbesondere durch einen Verdünnungsschritt, zu der gebrauchsfertigen Infusionslösung für die parenterale Verabreichung herrichtbar oder hergerichtet ist. Durch die Verdünnung, z.B. mit gegebenenfalls isotonisierter, wässriger Lösung, vorzugsweise mit physiologischer Kochsalzlösung, insbesondere in einem Verhältnis von 1:10 bis 1:500, vorzugsweise 1:20 bis 1:250, ist dann die gebrauchsfertige parenterale Infusionslösung erhältlich. Erfindungsgemäß ist dementsprechend auch eine gebrauchsfertige Infusionslösung vorgesehen, erhältlich durch Verdünnen der Zusammensetzung mit einer geeigneten Trägerlösung, insbesondere mit isotonisierter, wässriger Lösung, vorzugsweise mit physiologischer Kochsalzlösung, wässriger Glucoselösung oder isotonen Elektrolyt-Infusionslösungen, beispielsweise Ringerlösung oder Ringer-Lactat-Lösung, insbesondere in einem Verhältnis von 1:10 bis 1:500, vorzugsweise 1:20 bis 1:250.

Alternativ ist die Verwendung der Zusammensetzung als konzentrierte Injektionslösung für die direkte Verabreichung möglich.

Die Säure wird im Sinne der Erfindung vorzugsweise während des Herstellprozesses des Fertigarzneimittels dem Lösungsmittel zugegeben. Mögliche Ausführungsvarianten wären, die Säure während des Herstellprozesses des Lösungsmittels oder eines weiteren Hilfsstoffes oder während des Herstellprozesses des Wirkstoffes Alprostadil zuzugeben. Erfindungsgemäß ist dementsprechend in einem weiteren Aspekt ein Verfahren zur Herstellung einer flüssigen pharmazeutischen Zusammensetzung, als Konzentrat, vorgesehen, im Zuge dessen das Alprostadil in einem Gemisch aus Wasser und einem organischen Lösungsmittel gelöst wird und dieser Lösung zumindest eine Säure bzw. eine als Säure bzw. Protonendonator wirkende Substanz, wie zuvor beschrieben, als Stabilisator für den Wirkstoff zugesetzt wird.

Die in dieser Anmeldung angegebenen Konzentrationsangaben, z.B. für Alprostadil etc. beziehen sind auf ein solches Konzentrat und nicht auf die gebrauchsfertige, verdünnte Infusionslösung.

Die erfindungsgemäße Zusammensetzung dient in ihrer wichtigsten Indikation zur Behandlung der chronischen arteriellen Verschlusskrankheit im Stadium III und IV, insbesondere beim Menschen, insbesondere wenn eine Therapie zur Erweiterung der Blutgefäße nicht möglich oder erfolglos ist.

Erfindungsgemäß ist weiters die Verwendung zumindest einer Säure bzw. einer als Säure bzw. Protonendonator wirkenden Substanz, wie zuvor beschrieben, als Stabilisator für den Wirkstoff Alprostadil bzw. für eine, wie zuvor beschriebene, flüssige pharmazeutische Zusammensetzung, enthaltend den in Wasser und einem organischen Lösungsmittel gelösten Wirkstoff Alprostadil.

### Beispiele:

Im Folgenden wird die Erfindung an Hand von einigen exemplarischen aber nicht einschränkend zu verstehenden Ausführungsbeispielen dargestellt:
Vergleichsbeispiel 1:
(Laboransatz / 50 % Ethanol / 50 % Wasser / 20 µg/ml Alprostadil / Vials)

In einem 2,5 I Glasreaktor mit Rührwerk werden Ethanol absolut und Wasser vorgelegt, der Wirkstoff Alprostadil wird unter Rühren zugegeben, die Lösung wird in 2ml Typ I Braunglas Vials abgefüllt und die Vials werden mit einem Chlorbutyl-Kautschuk Stopfen verschlossen.

| | Menge in g |
|---|---|
| Alprostadil | 0,037 |
| Ethanol absolut | 850 |
| Wasser | 850 |

Beispiel 2:
(Laboransatz / 50 % Ethanol / 50 % Wasser / 20 µg/ml Alprostadil / 40 µg/ml Essigsäure / Vials)

In einem 2,5 I Glasreaktor mit Rührwerk werden Ethanol absolut und Wasser vorgelegt und Essigsäure wasserfrei wird zugegeben. Nach vollständigem Auflösen wird der Wirkstoff Alprostadil unter Rühren zugegeben, die Lösung in 2ml Typ I Braunglas Vials abgefüllt und die Vials werden mit einem Chlorbutyl-Kautschuk Stopfen verschlossen.

| | Menge in g |
|---|---|
| Alprostadil | 0,037 |
| Ethanol absolut | 850 |
| Wasser | 850 |
| Essigsäure, wasserfrei | 0,074 |

Beispiel 3:
(Laboransatz / 75 % Ethanol / 25 % Wasser / 20 µg/ml Alprostadil / 20 µg/ml Äpfelsäure / Vials)

In einem 2,5 I Glasreaktor mit Rührwerk werden Ethanol absolut und Wasser vorgelegt und DL-Äpfelsäure zugegeben. Nach vollständigem Auflösen wird der Wirkstoff Alprostadil unter Rühren zugegeben, die Lösung wird in 2ml Typ I Braunglas Vials abgefüllt und die Vials werden mit einem Chlorbutyl-Kautschuk Stopfen verschlossen.

| | Menge in g |
|---|---|
| Alprostadil | 0,040 |
| Ethanol absolut | 1275 |
| Wasser | 425 |
| DL-Äpfelsäure | 0,040 |

Beispiel 4:
(Laboransatz / 40 % Ethanol / 60 % Wasser / 20 µg/ml Alprostadil / 20 µg/ml Äpfelsäure / Fertigspritzen)

In einem 2,5 I Glasreaktor mit Rührwerk werden Ethanol absolut und Wasser vorgelegt und DL-Äpfelsäure zugegeben. Nach vollständigem Auflösen wird der Wirkstoff Alprostadil unter Rühren zugegeben und die Lösung wird in Fertigspritzen abgefüllt.

| | Menge in g |
|---|---|
| Alprostadil | 0,036 |
| Ethanol absolut | 680 |
| Wasser | 1020 |
| DL-Äpfelsäure | 0,036 |

Beispiel 5a:
(Laboransatz / 50 % Ethanol / 50 % Wasser / 20 µg/ml Alprostadil / 20 µg/ml Äpfelsäure / Ampullen)

In einem 2,5 I Glasreaktor mit Rührwerk werden Ethanol absolut und Wasser vorgelegt und DL-Äpfelsäure zugegeben. Nach vollständigem Auflösen wird der Wirkstoff Alprostadil unter Rühren zugegeben und die Lösung wird in Fertigspritzen abgefüllt.

| | Menge in g |
|---|---|
| Alprostadil | 0,037 |
| Ethanol absolut | 850 |
| Wasser | 850 |
| DL-Äpfelsäure | 0,037 |

Beispiel 5b:
(Produktionsansatz / 50 % Ethanol / 50 % Wasser / 20 µg/ml Alprostadil / 20 µg/ml DL-Äpfelsäure / Ampullen)

In einem 20 I Glasreaktor mit Rührwerk werden gekühlter Ethanol absolut und gekühltes Wasser vorgelegt und es wird DL-Äpfelsäure zugegeben. Nach vollständigem Auflösen wird der Wirkstoff Alprostadil unter Rühren zugegeben. Nach vollständigem Auflösen des Wirkstoffes wird die Lösung über einen Sterilfilter mit einer Porenweite von 0,2 µm filtriert und unter aseptischen Bedingungen in 2ml Typ I Braunglas Ampullen abgefüllt. Die abgefüllten Ampullen werden auf sichtbare Partikel und Dichtigkeit überprüft, etikettiert und sekundärverpackt.

| | Menge in g |
|---|---|
| Alprostadil | 0,345 |
| Ethanol absolut | 7890 |
| Wasser | 7890 |
| DL-Äpfelsäure | 0,345 |

Die Formulierungen gemäß den Beispielen 4, 5a und 5b stellen besonders bevorzugte Ausführungsformen der Erfindung dar.

Weitere Ausführungsbeispiele werden in den nachfolgenden Tabellen 3 und 4 aufgeführt, wobei jeweils nur die Zusätze zum Lösungsmittel bzw. Lösungsmittelgemisch in der Einheit µg/ml angegeben werden. Die Zusammensetzung des Lösungsmittels bzw. des Lösungsmittelgemisches selbst wird in Gew. % angegeben. Alle in den Tabellen aufgeführten Beispiele können in beliebiger Chargengröße hergestellt und in alle Arten geeigneter Packmittel abgefüllt werden.

**Tabelle 3: Rezepturbeispiele für Zubereitungen mit einer Alprostadil-Konzentration von 20 µg/ml**

| Beispiel | 6 | 7 | 8 | 9 | 10 | 11 | 12 | 13 | 14 |
|---|---|---|---|---|---|---|---|---|---|
| Ethanol absolut | | 80 | 40 | 75 | 50 | 20 | | | |
| Wasser | | 20 | 60 | 25 | 50 | 80 | 50 | 50 | 50 |
| Ethanol 96 % | 100 | | | | | | 50 | | 50 |
| Propylenglycol | | | | | | | | 50 | |
| | | | | | | | | | |
| PGE 1 | 20 | 20 | 20 | 20 | 20 | 20 | 20 | 20 | 20 |
| | | | | | | | | | |
| Essigsäure | | | | | | | | 50 | |
| HCl | | | | | | 0,5 | | | |
| Phosphorsäure | | | | 5 | | | | | |
| Äpfelsäure | | | | | 50 | | | | 50 |
| Acetylcystein | | | | | | | 20 | | |

**Tabelle 4: Rezepturbeispiele für Zubereitungen mit Alprostadil-Konzentrationen von 10 bis 1000 µg/ml**

| Beispiel | 15 | 16 | 17 | 18 | 19 | 20 | 21 | 22 | 23 |
|---|---|---|---|---|---|---|---|---|---|
| Ethanol absolut | 50 | | 50 | 50 | 40 | 90 | | | |
| Wasser | 50 | 25 | 50 | 50 | 60 | 10 | 50 | | 30 |
| Ethanol 96 % | | 75 | | | | | 50 | 100 | |
| Propylenglycol | | | | | | | | | 70 |
| | | | | | | | | | |
| PGE 1 | 10 | 10 | 500 | 500 | 500 | 1000 | 1000 | 500 | 500 |
| | | | | | | | | | |
| Essigsäure | | | | | | | | | 100 |
| HCl | | | | | 0,25 | 0,75 | | | |
| Phosphorsäure | | | | | | | 7,5 | | |
| Äpfelsäure | 20 | | | 20 | | | | 50 | |
| Acetylcystein | | | | 20 | | | | | |

Nachweis der stabilisierenden Wirkung des Säurezusatzes:
Der Nachweis der stabilisierenden Wirkung des Säurezusatzes wurde zunächst an rein alkoholischen Alprostadil Lösungen an Hand von Stabilitätsprüfungen bei der vorgeschriebenen Lagertemperatur von 4 °C und unter Stressbedingungen bei 25 °C / 65% RH geführt. Als Packmittel wurden jeweils 2 ml Vials aus Röhrenglas der hydrolytischen Klasse I verwendet. Diese Glasqualität ist identisch mit der Glasqualität für Ampullen. Nachfolgend wird nur über die Ergebnisse der Tests bei 25 °C berichtet, da diese Tests bereits nach kürzerer Lagerdauer Aussagen zu Unterschieden in der Stabilität erlauben. Aus langjährigen Stabilitätsprüfungen des Handelsproduktes Pridax^{®} 20 (Pridax 20 µg/ml Konzentrat zur Herstellung einer Infusionslösung) ist bekannt, dass die Abbaureaktionen in der Zubereitung bei 4 °C qualitativ in der gleichen Weise ablaufen wie bei 25 °C, allerdings etwa um den Faktor 10 langsamer.

In einem ersten, diese Erfindung konstituierenden Versuch wurde als Kontrolle die Formulierung des Handelsproduktes Pridax^{®} 20 µg/ml Konzentrat zur Herstellung einer Infusionslösung (Musterbezeichnung X03 130002-3), die als nächstkommender Stand der Technik zu betrachten ist, gegen eine Testformulierung, enthaltend 20 µg/ml Essigsäure und mit ansonsten gleicher Zusammensetzung (Musterbezeichnung X03 130002-9), geprüft.

Der Gehalt an PGE1 wird dabei in der Einheit (µg/ml) gemessen. In allen nachfolgenden Ergebnistabellen wird der Gehalt zur besseren Vergleichbarkeit in % des Ausgangswertes von PGE1 angegeben. Die Konzentration des Abbauproduktes PGA1 wird in Massenprozent (% m/m) bezogen auf den deklarierten Gehalt an PGE1 gemessen und angegeben.

Die nachfolgende Tabelle 5 zeigt für beide Formulierungen die Gehalte an PGE1 (Wirkstoff) und PGA1 (Abbauprodukt), jeweils direkt nach Herstellung (t = 0) und nach einer Lagerung über 12 Wochen bei 25° C / 65% RH (t = 12 w). Die Absolutwerte von PGE1 direkt nach Herstellung betrugen 18,3 µg/ml für die säurefreie Formulierung und 18,4 µg/ml für die essigsäurehaltige Formulierung.

**Tabelle 5:**

| Kontrolle vs 20 µg/ml Essigsäure, Lagerung 12 Wo bei 25° C | | | |
|---|---|---|---|
| Formulierung | | t = 0 | t = 12 Wochen |
| X03 130002-3 (ohne Säure) | PGE1 | 100 % | 84,2 % |
| | PGA1 | 0,28 % | 19,8 % |
| X03 130002-9 (mit 20 µg/ml Essigsäure) | PGE1 | 100 % | 95,6 % |
| | PGA1 | 0,13 % | 5,22 % |

Der Zusatz von 20 µg/ml Essigsäure zu der Alprostadil Lösung gemäß dem Stand der Technik führt nach 12 Wochen Lagerung bei 25 °C zu einem deutlichen Stabilisierungseffekt, erkennbar an einer signifikant geringeren Abnahme des Gehalts an PGE1 (Alprostadil Wirkstoff) und an einer signifikant geringeren Zunahme des Gehalts an PGA1 (Alprostadil Abbauprodukt).

In einem weiteren Versuch wurde dieser Test wiederum mit einer Alprostadil Lösung der Stärke 20 µg/ml wiederholt, wobei als Lösungsmittel ein Gemisch aus 50 Gew.-% Ethanol, wasserfrei und 50 Gew.-% Wasser und als Säure Äpfelsäure statt Essigsäure verwendet wurde.

Die nachfolgende Tabelle 6 zeigt für diese beiden Formulierungen die Gehalte an PGE1 (Wirkstoff) und PGA1 (Abbauprodukt), jeweils direkt nach Herstellung (t = 0) und nach einer Lagerung über 6 Wochen bei 25° C / 65% RH (t = 6 w). Die Absolutwerte von PGE1 direkt nach Herstellung betrugen 20,7 µg/ml für die säurefreie Formulierung und 21,4 µg/ml für die essigsäurehaltige Formulierung.

**Tabelle 6:**

| Kontrolle vs 20 µg/ml Äpfelsäure, Lagerung 6 Wo bei 25° C | | | |
|---|---|---|---|
| Formulierung | | t = 0 | t = 6 Wochen |
| X03 1500014-7 (50 % Wasser, ohne Säure) | PGE1 | 100 % | 92,0 % |
| | PGA1 | 0,56 % | 12,4 % |
| X03 1500014-2 (50 % Wasser, mit 20 µg/ml Äpfelsäure) | PGE1 | 100 % | 95,1 % |
| | PGA1 | 0,26 % | 6,15% |

Der Zusatz von 20 µg/ml Äpfelsäure führt nach 6 Wochen Lagerung bei 25 °C zu einem deutlichen Stabilisierungseffekt, erkennbar an einer signifikant geringeren Abnahme des Gehalts an PGE1 (Alprostadil Wirkstoff) und an einer signifikant geringeren Zunahme des Gehalts an PGA1 (Alprostadil Abbauprodukt). Der Wasserzusatz bedingt allerdings für beide Formulierungen eine Verschlechterung der Stabilität gegenüber den in der Tabelle 5 präsentierten Werten für die wasserfreie Formulierung.

Nachweis eines geeigneten Mischungsverhältnisses zwischen Wasser und dem organischen Lösungsmittel:
Um für die erfindungsgemäße Zubereitung den Einfluss des Wassers auf die Stabilität zu untersuchen, wurden Stabilitätsprüfungen bei 4 °C und bei 25 °C an ethanolischen Lösungen durchgeführt, die jeweils 20 µg/ml Alprostadil und Wassergehalte von 0 - 95 % enthielten.

Zur Beurteilung der erhaltenen, absoluten Stabilitätswerte wurden als Referenzwerte die Stabilitätskennzahlen auch für säurefreie Formulierungen mit unterschiedlichem Wassergehalten bestimmt. Graphik 2 zeigt für die Prüfung solcher säurefreien Formulierungen bei 25 °C, dass der Gehalt an Alprostadil bis zu einem Wasserzusatz von 50 Gew.% relativ stabil bleibt und sogar leicht ansteigt und erst bei höherem Wasserzusatz deutlich abnimmt. Der Gehalt an Verunreinigung A nimmt bis zu einem Wassergehalt von 50 Gew.% leicht ab, um dann bei höheren Wassergehalten deutlich anzusteigen.

Graphik 2 (Referenzbeispiel):

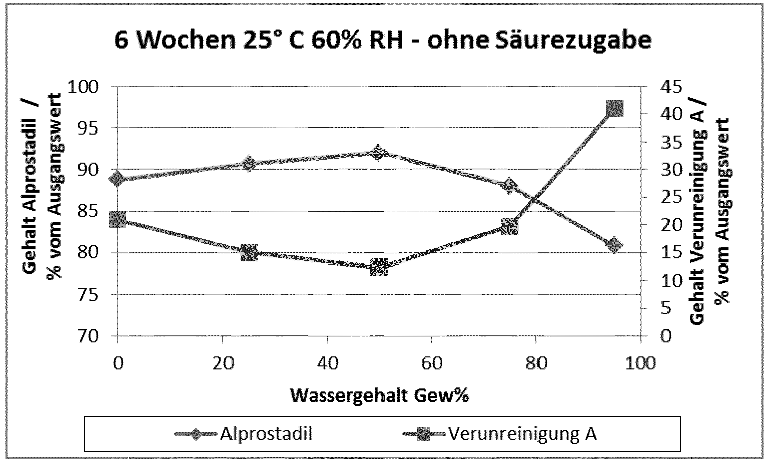

Als grundsätzlich mögliches Lösungsmittelgemisch hat sich somit für säurefreie Formulierungen insbesondere eine Mischung aus 50 Gew. % Ethanol und 50 Gew. % Wasser erwiesen, da bei diesem Mischungsverhältnis die Stabilität optimal und dem derzeitigen Stand der Technik ohne Wasserzusatz überlegen ist und da die medizinischen und technischen Vorteile einer Reduktion des Ethanols bereits signifikant sind.

Um für die erfindungsgemäße Zubereitung den Einfluss des Wassers auf die Stabilität zu untersuchen, wurden Stabilitätsprüfungen bei 4 °C und bei 25 °C an Lösungen durchgeführt, die jeweils 20 µg/ml Alprostadil, 20 µg/ml Äpfelsäure und Wassergehalte von 0 - 95 Gew. % enthielten.

Graphik 3 zeigt für die Prüfung bei 25 °C, dass der Gehalt an Alprostadil bis zu einem Wasserzusatz 50 % zwar abnimmt, aber relativ stabil bleibt und erst bei höherem Wasserzusatz deutlich abnimmt. Der Gehalt an Verunreinigung A nimmt mit steigendem Wasserzusatz bis zu einem Wassergehalt von 50 Gew. % leicht, ab einem Wassergehalt von 50 Gew. % sehr deutlich zu.

Graphik 3:

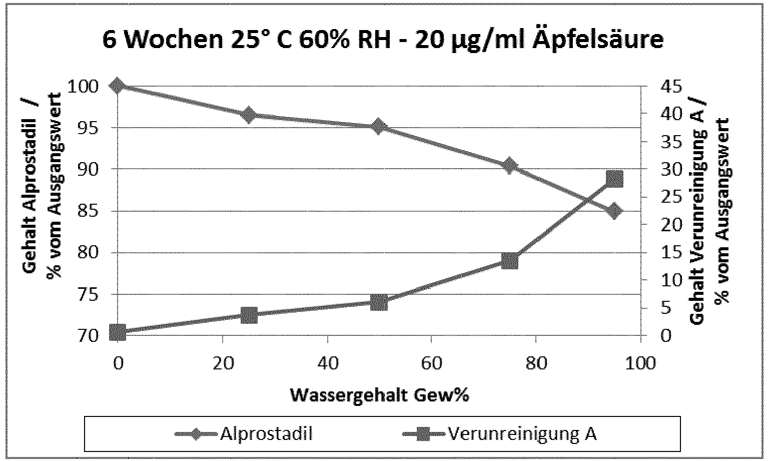

Ein im Sinne der Erfindung taugliches Lösungsmittel ist deshalb insbesondere eine Mischung enthalten ca. 45 % bis 55 Gew.% Ethanol, da bei diesem Mischungsverhältnis die Stabilität noch relativ gut und dem derzeitigen Stand der Technik ohne Wasser- und ohne Säurezusatz überlegen ist und da die medizinischen und technischen Vorteile einer Reduktion des Ethanols bereits signifikant sind.

Nachweis Einfluss der Menge bzw. Konzentration des Säurezusatzes:
In einem weiteren Versuch wurde, wiederum zunächst an der wasserfreien Formulierung, der Einfluss der Menge an Essigsäure auf den gefundenen Stabilisierungseffekt untersucht. Dazu wurde die Formulierung des Handelsproduktes Pridax^{®} 20 µg/ml Konzentrat zur Herstellung einer Infusionslösung (Musterbezeichnung X03 130007) gegen Testformulierungen, enthaltend 5 µg/ml, 20 µg/ml und 50 µg/ml Essigsäure und mit ansonsten gleicher Zusammensetzung (Musterbezeichnungen X03 130007-1, X03 130007-2 und X03 130007-1), geprüft.

Die nachfolgende Tabelle 7 sowie die dazugehörige Graphik 4 zeigen für diese vier Formulierungen die Gehalte an PGE1 (Wirkstoff) und PGA1 (Abbauprodukt), jeweils direkt nach Herstellung (t = 0) und nach einer Lagerung über 6 Wochen bei 25° C / 65% RH (t = 6 w). Die Absolutwerte von PGE1 direkt nach Herstellung betrugen zwischen 20,0 und 20,2 µg/ml.

**Tabelle 7:**

| Kontrolle vs Essigsäure in verschiedenen Konzentrationen, Lagerung 6 Wo bei 25° C | | | |
|---|---|---|---|
| Formulierung | | t = 0 | t = 6 Wochen |
| X03 130007 (ohne Säure) | PGE1 | 100 % | 93,5 % |
| | PGA1 | 0,13 % | 5,15 % |
| X03 130007-1 (mit 5 µg/ml Essigsäure) | PGE1 | 100 % | 95,0 % |
| | PGA1 | 0,12 % | 6,82 % |
| X03 130007-2 (mit 20 µg/ml Essigsäure) | PGE1 | 100 % | 97,0 % |
| | PGA1 | 0,10 % | 3,95 % |
| X03 130007-3 (mit 50 µg/ml Essigsäure) | PGE1 | 100 % | 99,0 % |
| | PGA1 | 0,11 % | 2,36 % |

Graphik 4:
Kontrolle vs Essigsäure in verschiedenen Konzentrationen, Lagerung 6 Wo bei 25° C

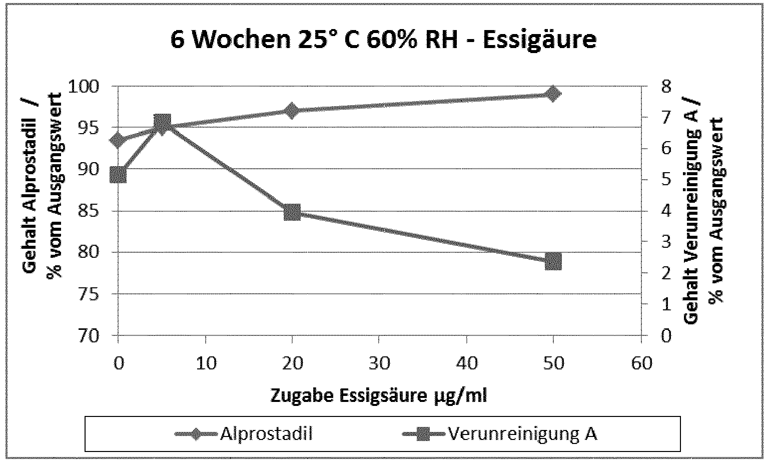

Die stabilisierende Wirkung des Zusatzes 20 µg/ml Essigsäure zu der Alprostadil Lösung konnte in diesem Versuch mittels der Probe X03 130007-2 reproduziert werden. Die Erhöhung der Essigsäuremenge auf 50 µg/ml führte zu einer weiteren Verbesserung der Stabilität, bei einer Konzentration von nur 5 µg/ml konnte kein signifikanter Stabilisierungseffekt beobachtet werden.

Die optimale Menge an Säure zur Stabilisierung einer wasserhaltigen Alprostadil Lösung, bei der als Säure Äpfelsäure statt Essigsäure verwendet wurde, wurde in einem weiteren Versuch experimentell bestimmt. Dazu wurde eine Formulierung, enthaltend 20 µg/ml Alprostadil in einem Lösungsmittelgemisch aus 50 Gew.-% Ethanol, wasserfrei und 50 Gew.-% Wasser mit 10, 20, 50, 100, 200, 500 und 0 µg/ml Äpfelsäure versetzt. (Musterbezeichnungen X03 1500014-1 bis X03 1500014-7) und hinsichtlich ihrer Stabilität geprüft.

Die nachfolgende Tabelle 8 sowie die dazugehörige Graphik 5 zeigen für diese sieben Formulierungen die Gehalte an PGE1 (Wirkstoff) und PGA1 (Abbauprodukt), jeweils direkt nach Herstellung (t = 0) und nach einer Lagerung über 6 Wochen bei 25° C / 65% RH (t = 6 w).

**Tabelle 8:**

| Kontrolle vs Äpfelsäure in verschiedenen Konzentrationen, Lagerung 6 Wo bei 25° C | | | |
|---|---|---|---|
| Formulierung | | t = 0 | t = 6 Wochen |
| X03 1500014-7 (50 % Wasser, ohne Säure) | PGE1 | 100 % | 92,0 % |
| | PGA1 | 0,56 % | 12,4 % |
| X03 1500014-1 (50 % Wasser, 10 µg/ml ÄS) | PGE1 | 100 % | 96,9 % |
| | PGA1 | 0,26 % | 6,3 % |
| X03 1500014-2 (50 % Wasser, 20 µg/ml ÄS) | PGE1 | 100 % | 95,1 % |
| | PGA1 | 0,26 % | 6,1 % |
| X03 1500014-3 (50 % Wasser, 50 µg/ml ÄS) | PGE1 | 100 % | 94,6 % |
| | PGA1 | 0,28 % | 6,5 % |
| X03 1500014-4 (50 % Wasser, 100 µg/ml ÄS) | PGE1 | 100 % | 94,2 % |
| | PGA1 | 0,29 % | 7,2 % |
| X03 1500014-5 (50 % Wasser, 200 µg/ml ÄS) | PGE1 | 100 % | 92,0 % |
| | PGA1 | 0,30 % | 8,6 % |
| X03 1500014-6 (50 % Wasser, 500 µg/ml ÄS) | PGE1 | 100 % | 88,8 % |
| | PGA1 | 0,39 % | 11,3 % |

Graphik 5:
Kontrolle vs Äpfelsäure in verschiedenen Konzentrationen, Lagerung 6 Wo bei 25° C

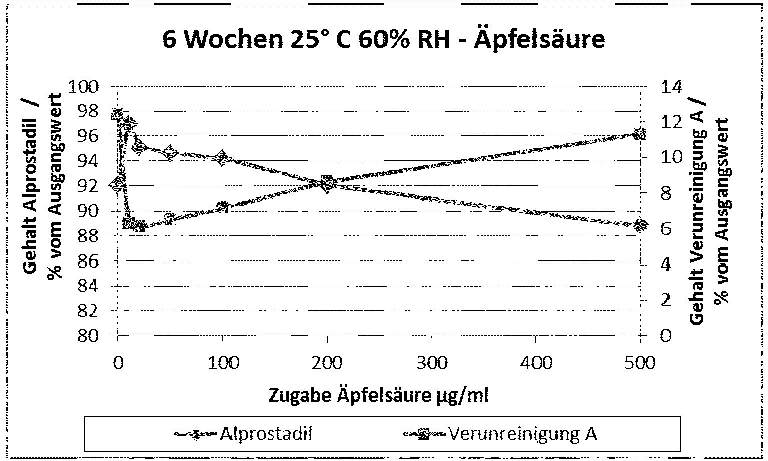

Die Daten zeigen, dass das Optimum der Stabilisierung bei einem Zusatz von 10 - 20 ppm Äpfelsäure eintritt. Diese Menge an Säure entspricht in etwa der Menge, die auch in der wasserfreien Lösung zu einer optimalen Stabilisierung führt.

Nachweis Art der Säure:
Die stabilisierende Wirkung verschiedener Säuren bei einem konstanten Säurezusatz von jeweils 20 µg/ml wurde vergleichend an einer wasserfreien Formulierung untersucht. Dazu wurde die Formulierung des Handelsproduktes Pridax^{®} 20 µg/ml Konzentrat zur Herstellung einer Infusionslösung (Musterbezeichnung X03 130007) gegen Testformulierungen, enthaltend jeweils 20 µg/ml Essigsäure (Musterbezeichnung X03 130007-2), Äpfelsäure (Musterbezeichnung X03 130007-5), Weinsäure (Musterbezeichnung X03 130007-8), Zitronensäure (Musterbezeichnung X03 130007-11), Acetylcystein (Musterbezeichnung X03 130007-14), Phosphorsäure (Musterbezeichnung X03 140006-6) und Salzsäure (Musterbezeichnung X03 140006-3) und mit ansonsten gleicher Zusammensetzung geprüft.

Die nachfolgende Tabelle 9 zeigt für diese acht Formulierungen die Gehalte an PGE1 (Wirkstoff) und PGA1 (Abbauprodukt), jeweils direkt nach Herstellung (t = 0) und nach einer Lagerung über 6 Wochen bei 25° C / 65% RH (t = 6 w). Die Absolutwerte von PGE1 direkt nach Herstellung betrugen zwischen 20,0 und 20,3 µg/ml.

**Tabelle 9:**

| Kontrolle vs 20 µg/ml verschiedener Säuren, Lagerung 6 Wo bei 25° C | | | |
|---|---|---|---|
| Formulierung | | t = 0 | t = 6 Wochen |
| X03 130007 (ohne Säure) | PGE1 | 100 % | 93,5 % |
| | PGA1 | 0,13 % | 5,15 % |
| X03 130007-2 (mit 20 µg/ml Essigsäure) | PGE1 | 100 % | 97,0 % |
| | PGA1 | 0,10 % | 3,95 % |
| X03 130007-5 (mit 20 µg/ml Äpfelsäure) | PGE1 | 100 % | 100,0 % |
| | PGA1 | < 0,05 % | 0,60 % |
| X03 130007-8 (mit 20 µg/ml Weinsäure) | PGE1 | 100 % | 98,0 % |
| | PGA1 | 0,06 % | 0,67 % |
| X03 130007-11 (mit 20 µg/ml Zitronensäure) | PGE1 | 100 % | 99,0 % |
| | PGA1 | 0,09 % | 0,50 % |
| X03 130007-14 (mit 20 µg/ml Acetylcystein) | PGE1 | 100 % | 98,0 % |
| | PGA1 | < 0,05 % | 0,71 % |
| X03 140006-6 (mit 20 µg/ml Phosphorsäure) | PGE1 | 100 % | 92,0 % |
| | PGA1 | 0,06 % | 13,56 % |
| X03 140006-3 (mit 20 µg/ml Salzsäure) | PGE1 | 100 % | 0 % |
| | PGA1 | < 0,05 % | < 0,05 |

Die dazugehörige Graphik 5 zeigt für die fünf Formulierungen, an denen eine stabilisierende Wirkung beobachtet wurde, und für die säurefreie Formulierung die Gehalte an PGE1 (Wirkstoff) und PGA1 (Abbauprodukt) nach einer Lagerung über 6 Wochen bei 25° C / 65% RH. Nicht dargestellt sind die Werte der Formulierungen mit Salzsäure und Phosphorsäure, da hier keine Stabilisierung beobachtet wurde. Der Zusatz von 20 µg/ml Salzsäure führt zu einem Totalabbau von Alprostadil und seinem Abbauprodukt PGA1, der Zusatz von 20 µg/ml Phosphorsäure zu einer Verschlechterung der Stabilität im Vergleich zu der säurefreien Formulierung. In beiden Fällen ist mit 20 µg/ml die für die Stabilisierung optimale Konzentration überschritten. Für Essigsäure beginnt die optimale Konzentration etwas höher, ab etwa 50 µg/ml.

### Graphik 5:

Kontrolle vs 20 µg/ml verschiedener Säuren, Lagerung 6 Wo bei 25° C

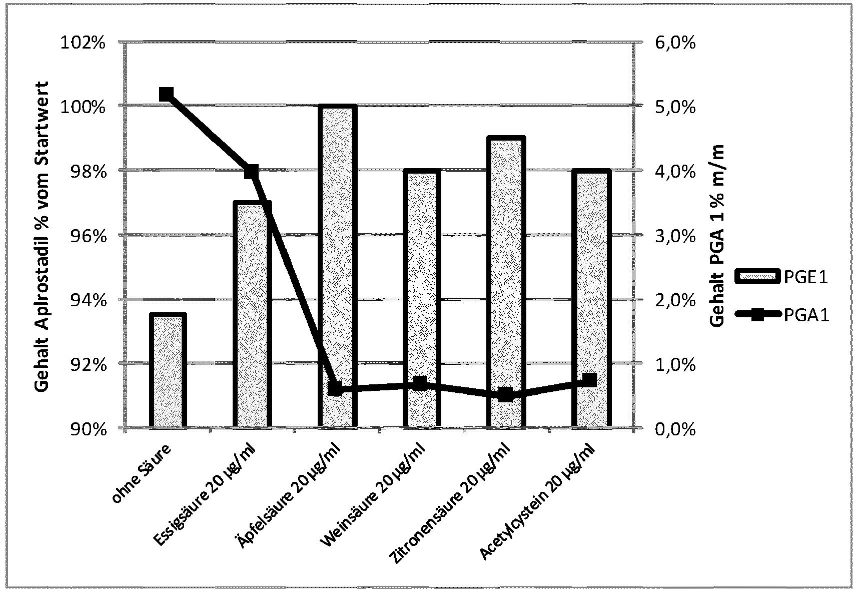

Der Versuch zeigt, dass der stabilisierende Effekt des Säurezusatzes in seinem Ausmaß von der Art der verwendeten Säure abhängt. Die zunächst verwendete Essigsäure hat einen geringeren Stabilisierungseffekt als mehrwertige organische Säuren und Acetylcystein. Für den Funktionsmechanismus des Stabilisierungseffekts bedeutet das, dass es sich nicht nur um eine unspezifische Optimierung der Säurekonzentration handelt, sondern dass überraschenderweise ein säurespezifischer Stabilisierungsmechanismus zu beobachten ist. Nachweis eines stabilisierenden Effektes auch der starken Säuren Phosphorsäure und Salzsäure:
Da für Phosphorsäure und Salzsäure bei einer Konzentration von 20 µg/ml kein stabilisierender Effekt auftrat, wurde in weiteren Versuchen geprüft, ob ein solcher Effekt für diese Säuren bei niedrigeren Konzentrationen nachweisbar ist.

Dazu wurde Phosphorsäure zusätzlich in einer Konzentration von 5 µg/ml sowohl in einer wasserfreien als auch in einer wasserhaltigen Formulierung geprüft. Die nachfolgende Tabelle 10 zeigt durch Vergleich mit der säurefreien Formulierung und einer Formulierung mit einem Zusatz von 20 µg/ml, dass bei der Konzentration von 5 µg/ml in gleicher Weise für die wasserfreie und die wasserhaltige Formulierung ein Stabilisierungseffekt auftritt:

**Tabelle 10:**

| Kontrolle vs 5 und 20 µg/ml Phosphorsäure, Lagerung 6 Wo bei 25° C | | | |
|---|---|---|---|
| Formulierung | | t = 0 | t = 6 Wochen |
| X03 130007 (wasserfrei, ohne Säure) | PGE1 | 100 % | 93,5 % |
| | PGA1 | 0,13% | 5,15% |
| X03 140006-5 (wasserfrei, mit 5 µg/ml Phosphorsäure) | PGE1 | 100 % | 98,5 % |
| | PGA1 | 0,08 % | 1,96 % |
| X03 1500016-02 (25 % Wasser, mit 5 µg/ml Phosphorsäure) | PGE1 | 100 % | 96,0 % |
| | PGA1 | 0,24 % | 5,0 % |
| X03 140006-6 (wasserfrei, mit 20 µg/ml Phosphorsäure) | PGE1 | 100 % | 92,0 % |
| | PGA1 | 0,06 % | 13,56 % |

Weiters wurde Salzsäure zusätzlich im Konzentrationsbereich von 0,05 µg/ml bis 2 µg/ml geprüft. Auch bei Salzsäure wurde ein stabilisierender Effekt in einem Konzentrationsbereich von 0,1 - 0,8 µg/ml beobachtet, das Optimum der Stabilisierung wurde für die wasserfreie Formulierung bei etwa 0,5 µg/ml beobachtet, bereits bei einem Zusatz von 2 µg/ml war die Stabilität signifikant schlechter als ohne Säurezusatz.

Nachweis Menge bzw. Konzentration an Äpfelsäure:
Da sich beim Vergleich der unterschiedlichen Säuren Äpfelsäure als besonders tauglich erwies und da Äpfelsäure wegen ihre toxikologischen Profils für die Verwendung in parenteralen Zubereitungen sehr gut geeignet ist, wurde in einem weiteren Versuch der Einfluss der Menge bzw. der Konzentration an Äpfelsäure auf den gefundenen Stabilisierungseffekt untersucht. Dazu wurde - analog zum oben mit Essigsäure beschriebenen Versuch - die Formulierung des Handelsproduktes Pridax^{®} 20 µg/ml Konzentrat zur Herstellung einer Infusionslösung (Musterbezeichnung X03 130007) gegen Testformulierungen, enthaltend 5 µg/ml, 20 µg/ml und 50 µg/ml DL-Äpfelsäure und mit ansonsten gleicher Zusammensetzung (Musterbezeichnungen X03 130007-4, X03 130007-5 und X03 130006-1), geprüft.

Die nachfolgende Tabelle 11 sowie die dazugehörige Graphik 6 zeigen für diese vier Formulierungen die Gehalte an PGE1 (Wirkstoff) und PGA1 (Abbauprodukt), jeweils direkt nach Herstellung (t = 0) und nach einer Lagerung über 6 Wochen bei 25° C / 65% RH (t = 6 w). Die Absolutwerte von PGE1 direkt nach Herstellung betrugen zwischen 20,0 und 20,1 µg/ml.

**Tabelle 11:**

| Kontrolle vs Äpfelsäure in verschiedenen Konzentrationen, Lagerung 6 Wo bei 25° C | | | |
|---|---|---|---|
| Formulierung | | t = 0 | t = 6 Wochen |
| X03 130007 (ohne Säure) | PGE1 | 100 % | 93,5 % |
| | PGA1 | 0,13 % | 5,15% |
| X03 130007-4 (mit 5 µg/ml DL-Äpfelsäure) | PGE1 | 100 % | 100,0% |
| | PGA1 | < 0,05 % | 1,19 % |
| X03 130007-5 (mit 20 µg/ml DL-Äpfelsäure) | PGE1 | 100 % | 100,0% |
| | PGA1 | < 0,05 % | 0,60 % |
| X03 130007-6 (mit 50 µg/ml DL-Äpfelsäure) | PGE1 | 100 % | 98,5 % |
| | PGA1 | < 0,05 % | 0,72 % |

Graphik 6:
Kontrolle vs Äpfelsäure in verschiedenen Konzentrationen, Lagerung 6 Wo bei 25° C

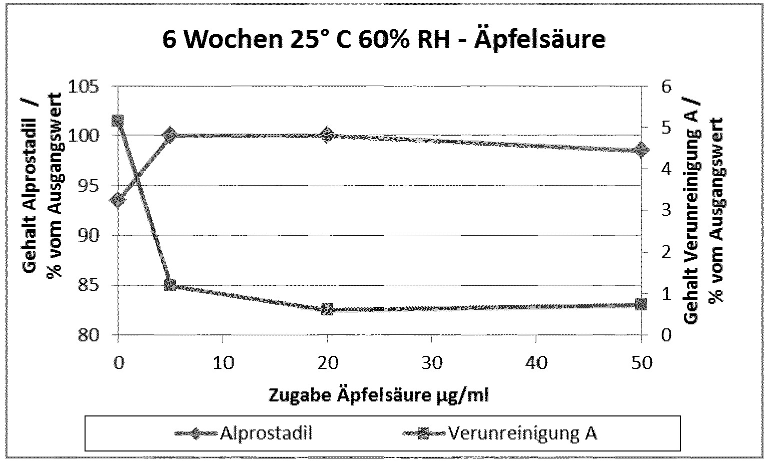

Der Versuch zeigt, dass Äpfelsäure das Alprostadil schon bei dem sehr geringen Zusatz von 5 µg/ml signifikant stabilisiert. Eine Erhöhung des Säurezusatzes auf 20 µg/ml führt zu einer Verbesserung des Stabilisierungseffekts, eine weitere Erhöhung auf 50 µg/ml führt dagegen zu keiner weiteren Verbesserung der Stabilität.

Für die wasserhaltigen Formulierungen wurde das Optimum der für einen Stabilisierungseffekt benötigten Äpfelsäuremenge bereits in der Tabelle 8 dargestellt. Es zeigt sich, dass sich durch den Zusatz von Wasser die benötigte Säuremenge nicht wesentlich ändert.

Zusammenfassung der Versuchsergebnisse zur Art und zur Menge der benötigten Säure:
Geeignet zur Stabilisierung von Alprostadil in den erfindungsgemäßen Lösungen sind solche Säuren, deren pKs1-Wert niedriger als der pKs-Wert von Alprostadil selbst, also niedriger als 4,85 ist, und die außer ihrer Acidität keine weiteren die Stabilität beeinflussenden Eigenschaften haben.

Für die geeigneten Säuren besteht ein Zusammenhang zwischen dem pKs1-Wert und der benötigten Säuremenge bzw. -konzentration in der Weise, dass bei sinkendem pKs1-Wert auch der Säurebedarf sinkt.

Aus den Stabilitätswerten kann durch exponentielle Regression annähernd folgender Zusammenhang zwischen dem pKs1-Wert der stabilisierenden Säure und der benötigten Säurekonzentration hergestellt werden:
C max in µM c= 11,75 e(1,35* pKs1)
C min in µM c = 1,45 e(1,02* pKs1)

Diese Formeln geben die zur Stabilisierung benötigte Säurekonzentration in der Einheit µM (= µmol / l) an. Sie gilt für geeignete organische und anorganische Säuren in einem pKs1-Bereich von 0 bis 4,85.

Für Säuren mit einem pKs1 < 0 wird dieser mit 0 angenommen, woraus sich folgende konstante Werte ergeben:
C max = 13,2 µM
C min = 1,3 µM

Dieser Zusammenhang gilt sowohl für wasserfreie als auch für die erfindungsgemäßen wasserhaltigen Formulierungen.

Die zuvor bereits beschriebene Tabelle 2 gibt beispielhaft zur Stabilisierung geeignete Konzentrationen für verschiedene Säuren in µg/ml an.

## Patentansprüche

1. Flüssige pharmazeutische Zusammensetzung in Form eines Konzentrats zur Herstellung einer Infusionslösung, enthaltend Alprostadil (Prostaglandin E1) als Wirkstoff, gelöst in einem Lösungsmittelgemisch aus zumindest einem pharmazeutisch annehmbaren organischen Lösungsmittel und Wasser, **dadurch gekennzeichnet, dass**
das organische Lösungsmittel ausgewählt ist aus der Gruppe von Propylenglycol, 1,3 Butandiol, Ethylacetat, Ethanol 96 % PhEur oder Wasserfreies Ethanol PhEur bzw. absoluter Ethanol oder Mischungen davon,
dass der Gesamt-Wasseranteil im Lösungsmittelgemisch im Bereich von 8 bis 85 Gew.% H₂O, bezogen auf die Gesamtmenge des Lösungsmittelgemisches, liegt,
und dass zumindest eine Säure bzw. eine als Säure bzw. Protonendonator wirkende Substanz als Stabilisator für den Wirkstoff enthalten ist, wobei die Säure einen pKs1-Wert von < 4,85 aufweist,
und dass die Zusammensetzung frei von Salzen ist

2. Zusammensetzung nach Anspruch 1, bestehend aus Alprostadil als Wirkstoff, gelöst in einem Lösungsmittelgemisch aus zumindest einem pharmazeutisch annehmbaren organischen Lösungsmittel, insbesondere Ethanol, und Wasser, wobei der Gesamt-Wasseranteil im Lösungsmittelgemisch im Bereich von 8 bis 85 Gew.% H₂O, bezogen auf die Gesamtmenge des Lösungsmittelgemisches, liegt und aus zumindest einer Säure bzw. einer als Säure bzw. Protonendonator wirkenden Substanz als Stabilisator für den Wirkstoff.

3. Zusammensetzung nach Anspruch 1 oder 2, **dadurch gekennzeichnet, dass** der Gesamt-Wasseranteil im Lösungsmittelgemisch im Bereich von 10 bis 80 Gew.% H₂O, vorzugsweise von 20 bis 60 Gew.% H₂O, insbesondere von 45 bis 55 Gew.% H₂O, bezogen auf die Gesamtmenge des Lösungsmittelgemisches, liegt.

4. Zusammensetzung nach einem der Ansprüche 1 bis 3, **dadurch gekennzeichnet, dass** die Zusammensetzung frei von weiteren Bestandteilen, Exzipienten oder Additiven, insbesondere frei von Isotonisierungszusätzen und/oder Cyclodextrinen, ist.

5. Zusammensetzung nach einem der Ansprüche 1 bis 4, **dadurch gekennzeichnet, dass** das, insbesondere jedes, organische Lösungsmittel, insbesondere vollständig, mit Wasser mischbar ist bzw. dass das Lösungsmittelgemisch aus dem zumindest einen organischen Lösungsmittel und Wasser eine echte einphasige Lösung ist.

6. Zusammensetzung nach einem der Ansprüche 1 bis 5, **dadurch gekennzeichnet, dass** Alprostadil in einer Konzentration von 1-1000 µg/ml, vorzugsweise 5-750 µg/ml, insbesondere 20-500 µg/ml enthalten ist.

7. Zusammensetzung nach einem der Ansprüche 1 bis 6, **dadurch gekennzeichnet, dass** die Säure eine organische Säure, insbesondere eine Carbonsäure, ist.

8. Zusammensetzung nach einem der Ansprüche 1 bis 7, **dadurch gekennzeichnet, dass** die Säure eine Hydroxycarbonsäure ist,
und/oder
dass die Säure aus der Gruppe Äpfelsäure, Essigsäure, Weinsäure, Milchsäure, Acetylcystein oder Zitronensäure ausgewählt ist,
und/oder
dass die Säure eine mehrwertige Hydroxycarbonsäure, vorzugsweise Zitronensäure, Äpfelsäure oder Weinsäure, ist.

9. Zusammensetzung nach einem der Ansprüche 1 bis 8, **dadurch gekennzeichnet, dass** die Säure Acetylcystein ist,
und/oder
dass die Säure Äpfelsäure, insbesondere DL- Äpfelsäure, ist.

10. Zusammensetzung nach einem der Ansprüche 1 bis 6, **dadurch gekennzeichnet, dass** die Säure eine anorganische Säure, insbesondere ausgewählt aus der Gruppe Salzsäure oder Phosphorsäure, ist.

11. Zusammensetzung nach einem der Ansprüche 1 bis 10, **dadurch gekennzeichnet, dass** die Säure einen pKs1-Wert im Bereich von 2 bis 4,8 aufweist.

12. Zusammensetzung nach einem der Ansprüche 1 bis 10, **dadurch gekennzeichnet, dass** die Säure einen pKs1-Wert von größer gleich 0 bis kleiner 4,85 aufweist und in einer Konzentration von größer gleich c min = 1,45 * e ^{(1,02 * pKs1)} µM (=µmol/l) und kleiner gleich c max = 11,75 * e ^{(1,35 * pKs1)} µM (=µmol/l) enthalten ist,
oder
dass die Säure einen pKs1-Wert von kleiner 0 aufweist und in einer Konzentration von größer gleich c min = 1,0 µM (=µmol/l) und kleiner gleich c max = 15 µM (=µmol/l), vorzugsweise größer gleich c min = 1,3 µM (=µmol/l) und kleiner gleich c max = 13,2 µM (=µmol/l), enthalten ist.

13. Zusammensetzung nach einem der Ansprüche 1 bis 12, **dadurch gekennzeichnet, dass** die Säure in einer Konzentration von 0,05-500 µg/ml, vorzugsweise 0,1-100 µg/ml, insbesondere 5-50 µg/ml, enthalten ist.

14. Zusammensetzung nach einem der Ansprüche 1 bis 13, **dadurch gekennzeichnet, dass** die Säure einen pKs1-Wert von größer gleich 2 bis kleiner gleich 4,8 aufweist und in einer Konzentration von 0,5 bis 500 µg/ml, insbesondere von 1 bis 250 µg/ml, enthalten ist, und/oder

15. Zusammensetzung nach einem der Ansprüche 1 bis 14, **dadurch gekennzeichnet, dass** die Säure Essigsäure ist und in einer Konzentration zwischen 20 und 500 µg/ml, insbesondere zwischen 50 und 250 µg/ml, enthalten ist,
oder
dass die Säure Acetylcystein ist und in einer Konzentration zwischen 5 und 350 µg/ml, insbesondere zwischen 10 und 100 µg/ml, enthalten ist,
oder
dass die Säure Äpfelsäure, insbesondere DL- Äpfelsäure, ist und in einer Konzentration zwischen 5 und 200 µg/ml, insbesondere zwischen 10 und 100 µg/ml, enthalten ist,
oder
dass die Säure Weinsäure ist und in einer Konzentration zwischen 2 und 150 µg/ml, insbesondere zwischen 5 und 50 µg/ml, enthalten ist,
oder
dass die Säure Phosphorsäure ist und in einer Konzentration zwischen 0,5 und 15 µg/ml, insbesondere zwischen 1 und 10 µg/ml, enthalten ist,
oder
dass die Säure Salzsäure ist und in einer Konzentration zwischen 0,1 und 0,8 µg/ml, insbesondere zwischen 0,3 und 0,7 µg/ml, enthalten ist.

16. Zusammensetzung nach einem der Ansprüche 1 bis 15, **dadurch gekennzeichnet, dass** diese ein Konzentrat zur Herstellung einer gebrauchsfertigen Infusionslösung für die parenterale Verabreichung, insbesondere durch einen Verdünnungsschritt mit einer geeigneten Trägerlösung, ist oder als Konzentrat zur weiteren Herstellung einer verdünnten, gebrauchsfertigen Infusionslösung für die parenterale Verabreichung hergerichtet ist.

17. Zusammensetzung nach einem der Ansprüche 1 bis 16, **dadurch gekennzeichnet, dass** diese in Glasampullen, Glasvials, Karpulen oder Fertigspritzen abgefüllt ist.

18. Kit-of-parts, umfassend die Zusammensetzung nach einem der Ansprüche 1 bis 17, enthalten in einer verschlossenen Glasampulle, Glasvial, Karpule oder Fertigspritze.

19. Zusammensetzung nach einem der Ansprüche 1 bis 18 zur Verwendung bei der Behandlung der peripheren arteriellen Verschlusskrankheit (pAVK), insbesondere mittels intravenöser Infusion.

20. Gebrauchsfertige Infusionslösung, erhältlich durch Verdünnen der Zusammensetzung nach einem der Ansprüche 1 bis 17 mit einer geeigneten Trägerlösung, insbesondere mit isotonisierter, wässriger Lösung, vorzugsweise mit physiologischer Kochsalzlösung, wässriger Glucoselösung oder isotonen Elektrolyt-Infusionslösungen, beispielsweise Ringerlösung oder Ringer-Lactat-Lösung, insbesondere in einem Verhältnis von 1:10 bis 1:500, vorzugsweise 1:20 bis 1:250.

21. Verwendung zumindest einer Säure bzw. einer als Säure bzw. Protonendonator wirkenden Substanz nach einem der vorangehenden Ansprüche als Stabilisator für den Wirkstoff Alprostadil oder für eine flüssige pharmazeutische Zusammensetzung nach einem der vorangehenden Ansprüche.

## Claims

1. Liquid pharmaceutical composition in the form of a concentrate for producing a solution for infusion, containing alprostadil (prostaglandin E1) as the active ingredient, dissolved in a solvent mixture consisting of at least one pharmaceutically acceptable organic solvent and water, **characterised in that**
the organic solvent is selected from the group consisting of propylene glycol, 1,3-butanediol, ethyl acetate, ethanol 96% PhEur or anhydrous ethanol PhEur or absolute ethanol or mixtures thereof,
**in that** the total water content in the solvent mixture is in the range from 8 to 85 wt.% H₂O, in relation to the total quantity of the solvent mixture,
and **in that** at least one acid or a substance acting as an acid or proton donor is present as a stabiliser for the active ingredient, wherein the acid has a pKs1 value of < 4.85, and **in that** the composition is free from salts.

2. Composition according to claim 1, consisting of alprostadil as the active ingredient, dissolved in a solvent mixture consisting of at least one pharmaceutically acceptable organic solvent, in particular ethanol, and water, wherein the total water content in the solvent mixture is in the range from 8 to 85 wt.% H₂O, in relation to the total quantity of the solvent mixture, and of at least one acid or a substance acting as an acid or proton donor as a stabiliser for the active ingredient.

3. Composition according to claim 1 or 2, **characterised in that** the total water content in the solvent mixture is in the range from 10 to 80 wt.% H₂O, preferably from 20 to 60 wt.% H₂O, in particular from 45 to 55 wt.% H₂O, in relation to the total quantity of the solvent mixture.

4. Composition according to any of claims 1 to 3, **characterised in that** the composition is free from further constituents, excipients or additives, in particular free from isotonisation additives and/or cyclodextrins.

5. Composition according to any of claims 1 to 4, **characterised in that** the, in particular each, organic solvent is, in particular completely, miscible with water or **in that** the solvent mixture consisting of the at least one organic solvent and water is a true single-phase solution.

6. Composition according to any of claims 1 to 5, **characterised in that** alprostadil is present at a concentration of 1-1000 µg/ml, preferably 5-750 µg/ml, in particular 20-500 µg/ml.

7. Composition according to any of claims 1 to 6, **characterised in that** the acid is an organic acid, in particular a carboxylic acid.

8. Composition according to any of claims 1 to 7, **characterised in that** the acid is a hydroxycarboxylic acid,
and/or
**in that** the acid is selected from the group consisting of malic acid, acetic acid, tartaric acid, lactic acid, acetylcysteine or citric acid,
and/or
**in that** the acid is a polyvalent hydroxycarboxylic acid, preferably citric acid, malic acid or tartaric acid.

9. Composition according to any of claims 1 to 8, **characterised in that** the acid is acetylcysteine,
and/or
**in that** the acid is malic acid, in particular DL-malic acid.

10. Composition according to any of claims 1 to 6, **characterised in that** the acid is an inorganic acid, in particular selected from the group consisting of hydrochloric acid or phosphoric acid.

11. Composition according to any of claims 1 to 10, **characterised in that** the acid has a pKs1 value in the range from 2 to 4.8.

12. Composition according to any of claims 1 to 10, **characterised in that** the acid has a pKs1 value from greater than or equal to 0 to less than 4.85 and is present at a concentration of greater than or equal to c min = 1.45 * e (102*PKs1) µM (=µmol/l) and less than or equal to c max = 11.75 * e (135*pKs1) µM (=µmοl/l),
or
**in that** the acid has a pKs1 value of less than 0 and is present at a concentration of greater than or equal to c min = 1.0 µM (=µmol/l) and less than or equal to c max = 15 µM (=µmol/l), preferably greater than or equal to c min = 1.3 µM (=nmol/l) and less than or equal to c max = 13.2 µM (=µmol/l).

13. Composition according to any of claims 1 to 12, **characterised in that** the acid is present at a concentration of 0.05-500 µg/ml, preferably 0.1-100 µg/ml, in particular 5-50 µg/ml.

14. Composition according to any of claims 1 to 13, **characterised in that** the acid has a pKs1 value from greater than or equal to 2 to less than or equal to 4.8 and is present at a concentration of 0.5 to 500 µg/ml, in particular of 1 to 250 µg/ml, and/or

15. Composition according to any of claims 1 to 14, **characterised in that** the acid is acetic acid and is present at a concentration of between 20 and 500 µg/ml, in particular between 50 and 250 µg/ml,
or
**in that** the acid is acetylcysteine and is present at a concentration of between 5 and 350 µg/ml, in particular between 10 and 100 µg/ml,
or
**in that** the acid is malic acid, in particular DL-malic acid, and is present at a concentration of between 5 and 200 µg/ml, in particular between 10 and 100 µg/ml,
or
**in that** the acid is tartaric acid and is present at a concentration of between 2 and 150 µg/ml, in particular between 5 and 50 µg/ml,
or
**in that** the acid is phosphoric acid and is present at a concentration of between 0.5 and 15 µg/ml, in particular between 1 and 10 µg/ml,
or
**in that** the acid is hydrochloric acid and is present at a concentration of between 0.1 and 0.8 µg/ml, in particular between 0.3 and 0.7 µg/ml.

16. Composition according to any of claims 1 to 15, **characterised in that** it is a concentrate for producing a ready-to-use solution for infusion for parenteral administration, in particular by means of a step of dilution with a suitable carrier solution, or is prepared as a concentrate to further produce a diluted, ready-to-use solution for infusion for parenteral administration.

17. Composition according to any of claims 1 to 16, **characterised in that** it is filled in glass ampoules, glass vials, cartridges or pre-filled syringes.

18. Kit-of-parts comprising the composition according to any of claims 1 to 17, contained in a sealed glass ampoule, glass vial, cartridge or pre-filled syringe.

19. Composition according to any of claims 1 to 18 for use in the treatment of peripheral arterial occlusive disease (PAOD), in particular by intravenous infusion.

20. Ready-to-use solution for infusion obtainable by diluting the composition according to any of claims 1 to 17 with a suitable carrier solution, in particular with isotonised aqueous solution, preferably with physiological saline solution, aqueous glucose solution or isotonic electrolyte solutions for infusion, for example Ringer's solution or Ringer's lactate solution, in particular in a ratio of 1:10 to 1:500, preferably 1:20 to 1:250.

21. Use of at least one acid or substance acting as an acid or proton donor according to any of the preceding claims as a stabiliser for the active ingredient alprostadil or for a liquid pharmaceutical composition according to any of the preceding claims.

## Revendications

1. Composition pharmaceutique liquide sous forme d'un concentré pour la préparation d'une solution de perfusion, contenant de l'alprostadil (prostaglandine E1) en tant que principe actif, dissous dans un mélange de solvants composé d'au moins un solvant organique pharmaceutiquement acceptable et de l'eau, **caractérisée en ce que**
le solvant organique est choisi parmi le groupe consistant en propylène glycol, 1,3-butandiol, acétate d'éthyle, éthanol à 96 % selon la PhEur ou éthanol anhydre selon la PhEur ou de l'éthanol absolu ou des mélanges de ceux-ci,
**en ce que** la teneur en eau totale dans le mélange de solvants se situe dans la plage allant de 8 à 85 % en poids d'H₂O, par rapport à la quantité totale du mélange de solvants, et **en ce qu'**elle contient au moins un acide ou une substance agissant comme acide ou donneur de protons en tant que stabilisateur pour le principe actif, dans laquelle l'acide présente une valeur pKs1 < 4,85,
et **en ce que** la composition est exempte de sels.

2. Composition selon la revendication 1, comprenant de l'alprostadil en tant que principe actif, dissous dans un mélange de solvants composé d'au moins un solvant organique pharmaceutiquement acceptable, en particulier de l'éthanol, et de l'eau, dans laquelle la teneur en eau totale dans le mélange de solvants se situe dans la plage allant de 8 à 85 % en poids d'H₂O, par rapport à la quantité totale du mélange de solvants, et d'au moins un acide ou une substance agissant comme acide ou donneur de protons en tant que stabilisateur pour le principe actif.

3. Composition selon la revendication 1 ou 2, **caractérisée en ce que** la teneur en eau totale dans le mélange de solvants se situe dans la plage allant de 10 à 80 % en poids d'H²O, de préférence de 20 à 60 % en poids d'H₂O, en particulier de 45 à 55 % en poids d'H²O, par rapport à la quantité totale du mélange de solvants.

4. Composition selon l'une quelconque des revendications 1 à 3, **caractérisée en ce que** la composition est exempte d'autres composants, excipients ou additifs, en particulier exempte d'additifs isotoniques et/ou cyclodextrines.

5. Composition selon l'une quelconque des revendications 1 à 4, **caractérisée en ce que**, en particulier chaque solvant organique peut, en particulier, être mélangé à de l'eau ou **en ce que** le mélange de solvants constitue, à partir de l'au moins un solvant organique et l'eau, une véritable solution monophasique.

6. Composition selon l'une quelconque des revendications 1 à 5, **caractérisée en ce que** l'alprostadil y est contenu en une concentration allant de 1 à 1000 µg/ml, de préférence de 5 à 750 µg/ml, en particulier de 20 à 500 µg/ml.

7. Composition selon l'une quelconque des revendications 1 à 6, **caractérisée en ce que** l'acide est un acide organique, en particulier un acide carboxylique.

8. Composition selon l'une quelconque des revendications 1 à 7, **caractérisée en ce que** l'acide est un acide hydroxycarboxylique,
et/ou
**en ce que** l'acide est choisi parmi le groupe consistant en acide malique, acide acétique, acide tartrique, acide lactique, acétylcystéine ou acide citrique, et/ou
**en ce que** l'acide est un acide hydroxycarboxylique polyvalent, de préférence l'acide citrique, l'acide malique ou l'acide tartrique.

9. Composition selon l'une quelconque des revendications 1 à 8, **caractérisée en ce que** l'acide est l'acétylcystéine,
et/ou
**en ce que** l'acide est l'acide malique, en particulier l'acide malique DL.

10. Composition selon l'une quelconque des revendications 1 à 6, **caractérisée en ce que** l'acide est un acide inorganique, en particulier choisi parmi le groupe consistant en l'acide chlorhydrique ou l'acide phosphorique.

11. Composition selon l'une quelconque des revendications 1 à 10, **caractérisée en ce que** l'acide présente une valeur pKs1 située dans la plage allant de 2 à 4,8.

12. Composition selon l'une quelconque des revendications 1 à 10, **caractérisée en ce que** l'acide présente une valeur pKs1 supérieure ou égale à 0 et inférieure à 4,85 et est contenu en une concentration supérieure ou égale à c min = 1,45* e (102*PKS1) |jM (=µmol/l) et inférieure ou égale à c max = 11,75* e ^{(1'35*PKS1)} µm (=µmοl/l),
ou
**en ce que** l'acide présente une valeur pKs1 inférieure à 0 et est contenu en une concentration supérieure ou égale à c min = 1,0 µm (=µmol/l) et inférieure ou égale à c max = 15 µm (=µmol/l), de préférence supérieure ou égale à c min = 1,3 µm (µmol/l) et inférieure ou égale à c max = 13,2 µm (µmol/l).

13. Composition selon l'une quelconque des revendications 1 à 12, **caractérisée en ce que** l'acide est contenu en une concentration allant de 0,05 à 500 µg/ml, de préférence de 0,1 à 100 µg/ml, en particulier de 5 à 50 µg/ml.

14. Composition selon l'une quelconque des revendications 1 à 13, **caractérisée en ce que** l'acide présente une valeur pKs1 supérieure ou égale à 2 et inférieure ou égale à 4,8 et est contenu en une concentration allant de 0,5 à 500 µg/ml, en particulier de 1 à 250 µg/ml, et/ou

15. Composition selon l'une quelconque des revendications 1 à 14, **caractérisée en ce que** l'acide est l'acide acétique et est contenu en une concentration comprise entre 20 et 500 µg/ml, en particulier entre 50 et 250 µg/ml,
ou
**en ce que** l'acide est l'acétylcystéine et est contenu en une concentration comprise entre 5 et 350 µg/ml, en particulier entre 10 et 100 µg/ml,
ou
**en ce que** l'acide est l'acide malique, en particulier l'acide malique DL, et est contenu en une concentration comprise entre 5 et 200 µg/ml, en particulier entre 10 et 100 | ig/ml,
ou
**en ce que** l'acide est l'acide tartrique et est contenu en une concentration comprise entre 2 et 150 µg/ml, en particulier entre 5 et 50 µg/ml,
ou
**en ce que** l'acide est l'acide phosphorique et est contenu en une concentration comprise entre 0,5 et 15 µg/ml, en particulier entre 1 et 10 µg/ml,
ou
**en ce que** l'acide est l'acide chlorhydrique et est contenu en une concentration comprise entre 0,1 et 0,8 µg/ml, en particulier entre 0,3 et 0,7 µg/ml.

16. Composition selon l'une quelconque des revendications 1 à 15, **caractérisée en ce que** celle-ci est un concentré pour la préparation d'une solution pour perfusion prête à l'emploi pour l'administration parentérale, en particulier par une étape de dilution avec une solution support appropriée, ou est conçue comme concentré pour la préparation ultérieure d'une solution pour perfusion diluée, prête à l'emploi pour l'administration parentérale.

17. Composition selon l'une quelconque des revendications 1 à 16, **caractérisée en ce que** celle-ci est conditionnée dans des ampoules en verre, des flacons en verre, des cartouches ou des seringues préremplies.

18. Kit de composants, comprenant la composition selon l'une quelconque des revendications 1 à 17, contenue dans une ampoule en verre, flacon en verre, cartouche ou seringue préremplie scellée.

19. Composition selon l'une quelconque des revendications 1 à 18 pour être utilisée dans le traitement de l'artériopathie périphérique oblitérante (APO), en particulier au moyen d'une perfusion par voie intraveineuse.

20. Solution pour perfusion prête à l'emploi, pouvant être obtenue en diluant la composition selon l'une quelconque des revendications 1 à 17 avec une solution support appropriée, en particulier avec une solution aqueuse, isotonique, de préférence avec une solution saline physiologique, une solution aqueuse de glucose ou des solutions pour perfusion d'électrolytes isotoniques, par exemple une solution de Ringer ou une solution de Ringer Lactate, en particulier selon un rapport de 1/10 à 1/500, de préférence de 1/20 à 1/250.

21. Utilisation d'au moins un acide ou d'une substance agissant comme acide ou donneur de protons selon l'une quelconque des revendications précédentes en tant que stabilisateur pour le principe actif alprostadil ou pour une composition pharmaceutique liquide selon l'une quelconque des revendications précédentes.
